# EUROPEAN PATENT APPLICATION

(11) **EP 4 721 660 A1**
(43) Date of publication of application: **08.04.2026**
(21) Application number: 26150990.5
(22) Date of filing: 28.01.2022
(51) Int. Cl.: A61B 5/145, G16H 15/00

(54) **THIRD PARTY ANALYTE MONITORING**

(30) Priority: 29.01.2021 US 202163143339 P
(62) Divisional of application: 22746715.6
(71) Applicant: Abbott Diabetes Care Inc., Alameda, California 94502 (US)
(72) Inventor: LEE, William Koo, Alameda, 94501 (US); REVOLTAR, Andrew, Burien, 98148 (US); KUMAR, Panganamala Ashwin, Oakland, 94618 (US); STRATIS, Steven, Orlando, 32828 (US); SWINEHART, Lindsey Colleen, Bend, 97707 (US)
(74) Representative: Mathys & Squire

(57) **Abstract**

Digital and graphical user interfaces for third party analyte monitoring applications are provided. For example, disclosed herein are various embodiments of methods, systems, and interfaces for connection interfaces, alarm notification settings interfaces, and logbook interfaces in a third party analyte monitoring application. In addition, various embodiments of interface enhancements are described, including enhanced visibility mode and interfaces for setting up a third party analyte monitoring application, among other embodiments.

## Description

### CROSS-REFERENCE TO RELATED APPLICATIONS

This application claims priority to U.S. Provisional Application No. 63/143,339, filed January 29, 2021, which is herein expressly incorporated by reference in its entirety for all purposes.

### FIELD

The subject matter described herein relates generally to third party analyte monitoring, as well as systems, methods, and devices relating thereto.

### BACKGROUND

The detection and/or monitoring of analyte levels, such as glucose, ketones, lactate, oxygen, hemoglobin A1C, or the like, can be vitally important to the health of an individual having diabetes. Patients suffering from diabetes mellitus can experience complications including loss of consciousness, cardiovascular disease, retinopathy, neuropathy, and nephropathy. Diabetics are generally required to monitor their glucose levels to ensure that they are being maintained within a clinically safe range, and may also use this information to determine if and/or when insulin is needed to reduce glucose levels in their bodies, or when additional glucose is needed to raise the level of glucose in their bodies.

Growing clinical data demonstrates a strong correlation between the frequency of glucose monitoring and glycemic control. Despite such correlation, however, many individuals diagnosed with a diabetic condition do not monitor their glucose levels as frequently as they should due to a combination of factors including convenience, testing discretion, pain associated with glucose testing, and cost.

To increase patient adherence to a plan of frequent glucose monitoring, in vivo analyte monitoring systems can be utilized, in which a sensor control device may be worn on the body of an individual who requires analyte monitoring. To increase comfort and convenience for the individual, the sensor control device may have a small form-factor and can be applied by the individual with a sensor applicator. The application process includes inserting at least a portion of a sensor that senses a user's analyte level in a bodily fluid located in a layer of the human body, using an applicator or insertion mechanism, such that the sensor comes into contact with the bodily fluid. The analyte monitoring system may also be configured to transmit analyte data, alarms, and alarm information to another device, from which a third party such as, for example, a caregiver, a parent, or a spouse, can review the data, receive such alarms, and/or take an appropriate action in response.

Despite their advantages, however, some third parties are reluctant to use analyte monitoring systems for various reasons, including the complexity and volume of data presented, a learning curve associated with the software and user interfaces for analyte monitoring systems, and an overall paucity of actionable information presented.

Thus, needs exist for digital and graphical user interfaces for third party analyte monitoring, as well as systems, methods and devices relating thereto, that are robust, user-friendly, and provide for timely and actionable responses.

### SUMMARY

Provided herein are example embodiments of digital and graphical user interfaces for third party analyte monitoring. In particular, described herein are systems, methods, and interfaces relating to an analyte monitoring application on a third party's display device for the monitoring of analyte-related information for one or more monitored users, wherein the third party can be a caregiver, a parent, a spouse, or any other individual, party, or entity interested in monitoring one or more users wearing sensor control devices.

According to one embodiment, various connection interfaces are provided for a third party analyte monitoring application. According to one example embodiment, a single connection interface can comprise a profile card section (with the monitored user's name, a current analyte level value, and a directional trend arrow), and an analyte graph section (with an analyte trend line, a shaded area to indicate a target analyte range, and alarm threshold lines). In other embodiments, a multiple connection interface is provided, comprising a plurality of profile cards, each of which is associated with a monitored user.

According to another embodiment, various alarm notification settings interfaces for a third party analyte monitoring application are provided. According to one aspect of the embodiments, the third party analyte monitoring application can be configured to display one or more alarm notification settings interfaces based on sensor type information about a monitored user's sensor control device that is received from a trusted computer system. In some embodiments, for example, a first set of alarm notification settings interfaces can be displayed based on a determination that the monitored user has a sensor control device that is a first sensor type, and wherein the first set of alarm notification settings interfaces can include a glucose readings setting. In some embodiments, a second set of alarm notification settings interfaces can be displayed based on a determination that the monitored user has a sensor control device that is a second sensor type, and wherein the second set of alarm notification settings interfaces can include a no recent data setting.

According to another embodiment, various logbook interfaces for a third party analyte monitoring application are provided. According to one aspect of the embodiments, the third party analyte monitoring application can be configured to display a logbook interface comprising a plurality of logbook entries grouped by date for a predetermined period of time. In many of the embodiments, the logbook interface can include a first logbook entry comprising an analyte level value, a directional trend arrow, and a time stamp. In some embodiments, a second logbook entry can comprise a textual indication that an analyte level of the monitored user is above a reportable analyte level upper limit. In some embodiments, another logbook entry can comprise a low glucose alarm or a high glucose alarm. In still other embodiments, a logbook entry can comprise an entry manually inputted by the monitored user.

Many of the embodiments provided herein are improved GUIs or GUI features for a third party analyte monitoring application, that are highly intuitive, user-friendly, and provide for rapid access to important physiological information of the monitored user. More specifically, these embodiments allow a third party to easily navigate through and between different user interfaces that can quickly indicate to the third party various physiological conditions of the monitored user, without requiring the third party to go through the arduous task of examining large volumes of data. Furthermore, some of the GUIs and GUI features and interfaces provide for versatility in that they allow for third parties to simultaneously monitor users having different models and versions of sensor control devices. Other improvements and advantages are provided as well. The various configurations of these devices are described in detail by way of the embodiments which are only examples.

Other systems, devices, methods, features and advantages of the subject matter described herein will be or will become apparent to one with skill in the art upon examination of the following figures and detailed description. It is intended that all such additional systems, devices, methods, features, and advantages be included within this description, be within the scope of the subject matter described herein, and be protected by the accompanying claims. In no way should the features of the example embodiments be construed as limiting the appended claims, absent express recitation of those features in the claims.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is a system overview of an analyte monitoring system comprising a sensor applicator, a sensor control device, a reader device, a network, a trusted computer system, and a local computer system.
FIG. 2A is a block diagram depicting an example embodiment of a reader device.
FIGS. 2B and 2C are block diagrams depicting example embodiments of sensor control devices.
FIG. 3A is an overview of a system for third party analyte monitoring, the system comprising a sensor control device, a monitored user's reader device, a network, a trusted computer system, and a third party's display device.
FIG. 3B is an overview of another system for third party analyte monitoring, the system comprising multiple sensor control devices, multiple monitored users' reader devices, a network, a trusted computer system, and multiple third parties' display devices.
FIGS. 4A to 4I are example embodiments of GUIs for an analyte monitoring application for enabling and/or disabling third party analyte monitoring.
FIG. 4J is a flow diagram depicting an example embodiment of a method for setting up third party analyte monitoring between a monitored user and a third party.
FIGS. 5A to 5S are example embodiments of GUIs relating to the setup of a third party analyte monitoring application.
FIG. 6A is a flow diagram depicting an example embodiment of a method for displaying a connection interface in a third party analyte monitoring application.
FIGS. 6B to 6C are example embodiments of GUIs for a third party analyte monitoring application relating to establishing a connection with a monitored user.
FIG. 7 is an example embodiment of a single connection interface for a third party analyte monitoring application, including an analyte graph section.
FIGS. 8A to 8J are example embodiments of connection interfaces for a third party analyte monitoring application including various connection interfaces for multiple monitored users.
FIGS. 9A to 9E are additional example embodiments of connection interfaces for a third party analyte monitoring application.
FIGS. 10A to 10H are example embodiments of GUIs depicting various logbook interfaces for a third party analyte monitoring application.
FIG. 10I is a flow diagram depicting an example embodiment of a method for displaying alarm notification settings interfaces for a third party analyte monitoring application.
FIGS. 11A to 11H are example embodiments of alarm notification settings interfaces for a third party analyte monitoring application.
FIGS. 12A to 12B are additional example embodiments of glucose readings notification settings interfaces for a third party analyte monitoring application.
FIGS. 13A to 13H are additional example embodiments of alarm notification settings interfaces for a third party analyte monitoring application.
FIGS. 14A to 14G are additional example embodiments of alarm notification settings interfaces for a third party analyte monitoring application.
FIGS. 15A to 15D are additional example embodiments of alarm notification settings interfaces for a third party analyte monitoring application.
FIGS. 15E to 15H are additional example embodiments of alarm notification settings interfaces for a third party analyte monitoring application.
FIGS. 16A to 16H are example embodiments of alarm notification interfaces for a third party analyte monitoring application.
FIGS. 17A to 17O are example embodiments of various interfaces for a third party analyte monitoring application.
FIGS. 18A to 18C are example embodiments of a menu interface and other interfaces for a third party analyte monitoring application.
FIGS. 19A to 19N are example embodiments of various interfaces in an enhanced visibility mode for a third party analyte monitoring application.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include GUIs and digital interfaces for third party analyte monitoring, and systems, methods, and devices relating thereto. Accordingly, many embodiments include in vivo analyte sensors structurally configured so that at least a portion of the sensor is, or can be, positioned in the body of a monitored user to obtain information about at least one analyte of the monitored user's body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices, reader devices, display devices, local computer systems, and trusted computer systems are disclosed, and these devices and systems can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps.

As previously described, a number of embodiments described herein provide for improved GUIs for a third party analyte monitoring application residing, for example, in memory of a caregiver's reader device, wherein the GUIs are actionable, user-friendly, and provide for rapid access to physiological information of a monitored user. According to some embodiments, for examples, methods and interfaces are provided for connection interfaces in a third party analyte monitoring application, wherein the connection interfaces can be configured to provide real-time (or near real-time) analyte information and interactive interfaces. According to other embodiments, methods and systems are provided for alarm notification settings interfaces for a third party analyte monitoring application with certain features that are enabled based on the sensor type utilized by the monitored user. In this regard, the third party analyte monitoring application can be both flexible and robust to simultaneously support multiple monitored users wearing different types and versions of sensor control devices. Additional improved digital and user interfaces for an analyte monitoring software application are described.

Collectively and individually, these methods, systems, and digital and user interfaces improve upon the usability and flexibility of third party analyte monitoring by allowing caregivers to receive improved information and interfaces about a monitored user's condition, as well as enhancing the alarming capabilities of the analyte monitoring systems. Other improvements and advantages are provided as well. The various configurations of these devices are described in detail by way of the embodiments which are only examples.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the monitored user, which can be analyzed to determine the monitored user's blood sugar level.

In vivo monitoring systems can include a sensor that, while positioned in vivo, makes contact with the bodily fluid of the monitored user and senses the analyte levels contained therein. The sensor can be part of the sensor control device that resides on the body of the monitored user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device, and processes and/or displays that sensed analyte data, in any number of forms, to a monitored user or a third party interested in monitoring the analyte levels of the monitored user (e.g., a caregiver, parent, spouse, or healthcare provider). This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, a "display" device, to name a few. Other devices such as personal computers have also been utilized with or incorporated into in vivo and in vitro monitoring systems.

### Example Embodiment of In Vivo Analyte Monitoring System

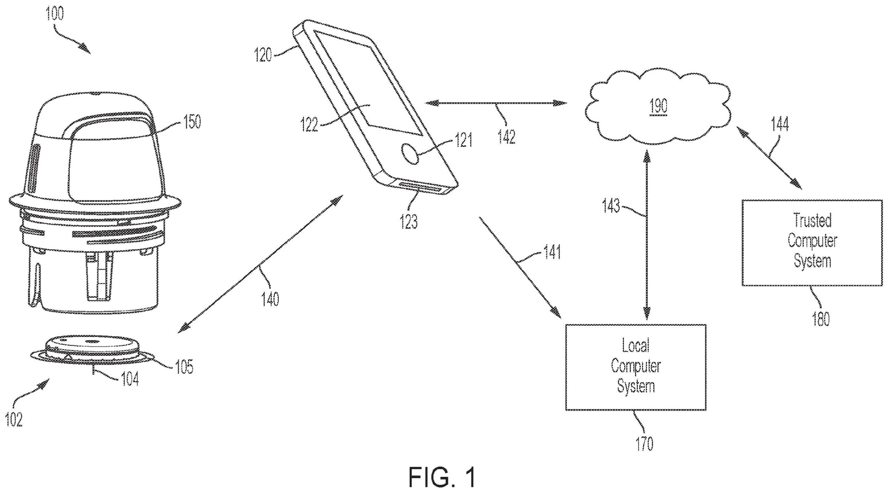

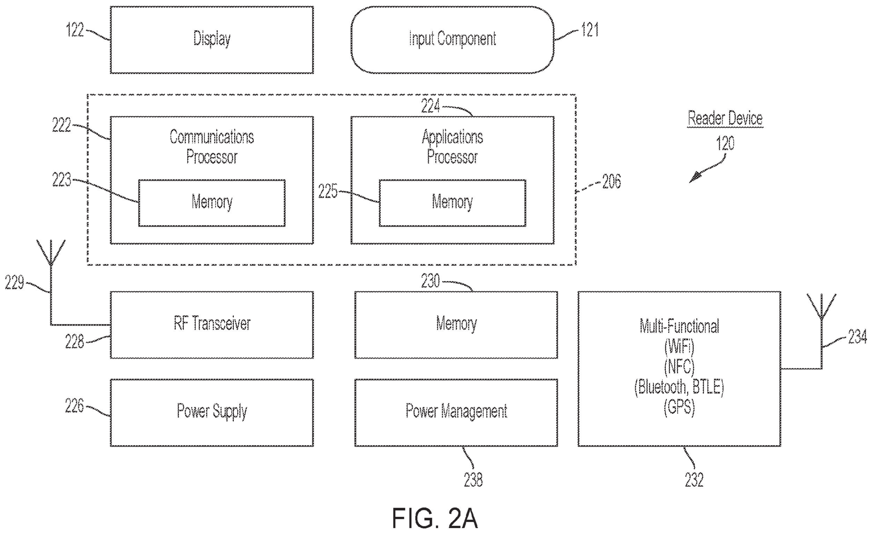

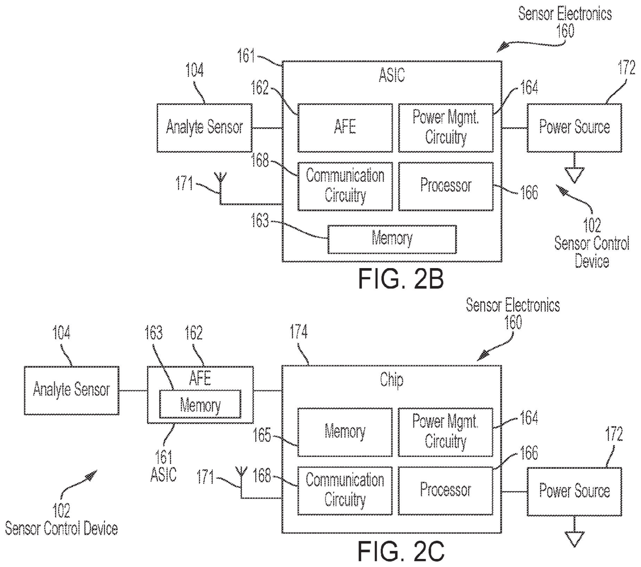

FIG. 1 is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a monitored user's skin where a sensor 104 is maintained in position for a period of time by an adhesive patch 105. Sensor control device 102 is further described in FIGS. 2B and 2C, and can communicate with reader device 120 via a communication path 140 using a wired or wireless technique. Example wireless protocols include Bluetooth, Bluetooth Low Energy (BLE, BTLE, Bluetooth SMART, etc.), NFC, and others. The monitored user can view and use applications installed in memory on reader device 120 using screen 122 (which, in many embodiments, can comprise a touchscreen), and input 121. A device battery of reader device 120 can be recharged using power port 123. While only one reader device 120 is shown, sensor control device 102 can communicate with multiple reader devices 120. Each of the reader devices 120 can communicate and share data with one another. More details about reader device 120 is set forth with respect to FIG. 2A below. Reader device 120 can communicate with local computer system 170 via a communication path 141 using a wired or wireless communication protocol. Local computer system 170 can include one or more of a laptop, desktop, tablet, phablet, smart phone, smart watch, wearable electronic device, fitness tracker, set-top box, video game console, or other computing device, and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by a wired or wireless communication protocol as described previously. Network 190 can be any of a number of networks, such as private networks and public networks, local area or wide area networks, and so forth. A trusted computer system 180 can include a cloud-based platform or server, and can provide for authentication services, secured data storage, report generation, and can communicate via communications path 144 with network 190 by wired or wireless technique. In addition, although FIG. 1 depicts trusted computer system 180 and local computer system 170 communicating with a single sensor control device 102 and a single reader device 120, it will be appreciated by those of skill in the art that local computer system 170 and/or trusted computer system 180 are each capable of being in wired or wireless communication with a plurality of reader devices and sensor control devices.

### Example Embodiment of Reader Device

FIG. 2A is a block diagram depicting an example embodiment of a reader device 120, which, in some embodiments, can comprise a smart phone. In some embodiments, reader device 120 can comprise a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further, reader device 120 can also include a multi-functional transceiver 232, which can comprise wireless communication circuitry, and which can be configured to communicate over Wi-Fi, NFC, Bluetooth, BTLE, and GPS with an antenna 234. As understood by one of skill in the art, these components are electrically and communicatively coupled in a manner to make a functional device.

### Example Embodiments of Sensor Control Devices

FIGS. 2B and 2C are block diagrams depicting example embodiments of sensor control devices 102 having analyte sensors 104 and sensor electronics 160 (including analyte monitoring circuitry) that can have the majority of the processing capability for rendering end-result data suitable for display to the monitored user or a third party. In FIG. 2B, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or non-volatile memory. In this embodiment, ASIC 161 is coupled with power source 172, which can be a coin cell battery, or the like. AFE 162 interfaces with in vivo analyte sensor 104 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to reader device 120 (not shown), for example, where minimal further processing is needed by the resident software application to display the data. According to some embodiments, for example, a current glucose value can be transmitted from sensor control device 102 to reader device 120 every minute, and historical glucose values can be transmitted from sensor control device 102 to reader device 120 every five minutes.

In some embodiments, data acquired from sensor control device 102 can be stored on reader device 120. According to one aspect of some embodiments, such data can include the model number and serial number of sensor control device 102, as well as information relating to the sensor control device 102's status, market code, or network address. In some embodiments, such data can also include error events detected by sensor control device 102. In addition, in some embodiments, either or both of current glucose values and historical glucose values can include one or more time stamps (e.g., factory time, UTC time, user's local time based on time zone, and the current time zone).

In some embodiments, sensor control device 102 can store data such that if reader device 120 is not in communication with sensor control device 102 (e.g., if reader device 120 is out of a wireless communication range, is powered off, or is otherwise unable to communicate with sensor control device 102), when reader device 120 re-establishes communication with sensor control device 102, data can then be backfilled to reader device 120. According to some embodiments, data that can be backfilled can include, but is not limited to, current and historical glucose values, as well as error events. Further details regarding data backfilling can be found in U.S. Patent No. 10,820,842, as well as U.S. Publ. No. 2021/0378601A1, both of which are hereby incorporated by reference in their entireties for all purposes.

According to some embodiments, each current glucose value and/or historical glucose value acquired from sensor control device 102 can further be validated on reader device 120, such as, for example, by performing a CRC integrity check to ensure that the data has been transferred accurately. In some embodiments, for example, a data quality mask of the current glucose value and/or historical glucose value can be checked to ensure that the reading is correct and can be displayed as a valid reading on the reader device 120.

According to another aspect of some embodiments, reader device 120 can include a database for storing any or all of the aforementioned data. In some embodiments, the database can be configured to retain data for a predetermined period of time (e.g., 30 days, 60 days, 90 days, six months, one year, etc.). According to some embodiments, the database can be configured to delete data after it has been uploaded to a cloud server. In other embodiments, database can be configured for a clinical setting, in which data is retained for a longer period of time (e.g., one year) relative to a non-clinical setting. In addition to the aforementioned data (e.g., current and/or historical glucose values, error events, etc.), the database on reader device 120 can also store user configuration information (e.g., login ID, notification settings, regional settings, and other preferences), as well as application configuration information (e.g., cloud settings, URLs for uploading data and/or error events, version information, etc.). The database can be encrypted to prevent a user from inspecting the data content directly even if the operating system of reader device 120 is compromised.

In some embodiments, to conserve power and processing resources on sensor control device 102, digital data received from AFE 162 can be sent to reader device 120 (not shown) with minimal or no processing. In still other embodiments, processor 166 can be configured to generate certain predetermined data types (e.g., current glucose value, historical glucose values) either for storage in memory 163 or transmission to reader device 120 (not shown), and to ascertain certain alarm conditions (e.g., sensor fault conditions), while other processing and alarm functions (e.g., high/low glucose threshold alarms) can be performed on reader device 120. Those of skill in the art will understand that the methods, functions, and interfaces described herein can be performed - in whole or in part -- by processing circuitry on sensor control device 102, reader device 120, local computer system 170, or trusted computer system 180.

FIG. 2C is similar to FIG. 2B but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 may include memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

### Example Embodiments of Systems for Third Party Analyte Monitoring

Described herein are example embodiments of systems, methods, and devices for third party analyte monitoring. As an initial matter, the term, "third party," can refer to an individual, an entity, or any party other than the monitored user, who is interested in monitoring the analyte levels of the monitored user, and information relating thereto. In some embodiments, for example, a third party can be a caregiver, a parent, or a spouse, and the monitored user can be an elder, a child, a spouse, or a patient. According to one aspect, the embodiments described herein include systems, methods, and devices similar to those described with respect to analyte monitoring system 100, but also include one or more "secondary" display devices utilized by third parties for monitoring the analyte levels of one or more monitored users. The secondary display device of a third party can comprise the same or similar componentry as reader device 120 of the monitored user, as described above, including one or more processors coupled with a memory for storing instructions that, when executed by the one or more processors, cause the one or more processors to execute a third party analyte monitoring application configured to monitor analyte levels of one or more monitored users.

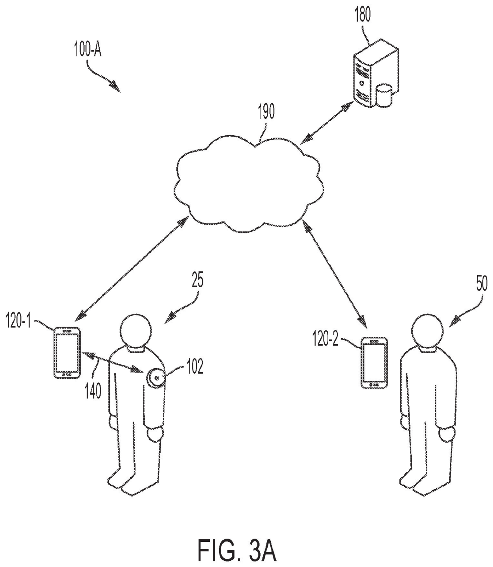

FIG. 3A is a conceptual diagram depicting an example embodiment of a system 100-A capable of communicating analyte data and other related information of a monitored user to a third party. According to one aspect of the embodiments, system 100-A shares a number of similarities with analyte monitoring system 100, as described with respect to FIG. 1. In particular, system 100-A can include a sensor control device 102, worn by monitored user 25, in wireless communication with reader device 120-1 via communication link 140. According to many of the embodiments, for example, sensor control device 102 can include communication circuitry configured to wirelessly communicate data with reader device 120-1 via a Bluetooth communication protocol or an NFC protocol. Furthermore, according to some embodiments, reader device 120-1 can be a smart phone, smart watch, wearable electronic device, dedicated receiver device, or glucose meter. Although one reader device 120-1 is depicted in FIG. 3A, monitored user 25 can have multiple reader devices (e.g., smart phones, glucose meters, dedicated receivers), each of which can be used to acquire analyte data from sensor control device 102. Furthermore, reader device 120-1 can be in wireless communication with trusted computer system 180 through network 190. In some embodiments, reader device 120-1 can include communication circuitry configured to wirelessly communicate data with trusted computer system 180 via an 802.11x communication protocol or a cellular communication protocol.

Referring still to FIG. 3A, analyte monitoring system 100-A further comprises a secondary display device 120-2 belonging to third party 50. As previously described, third party 50 can be a caregiver, parent, spouse, or any individual, party, or entity interested in monitoring the analyte levels of monitored user 25. Secondary display device 120-2 can include communication circuitry configured to wirelessly communicate data with trusted computer system 180 through network 190 via an 802.11x communication protocol or a cellular communication protocol. According to many embodiments, network 190 can be the Internet.

As described earlier, in many embodiments, sensor control device 102, which is worn by monitored user 25, includes an analyte sensor, at least a portion of which is configured to be positioned in the body of monitored user 25 and sense an in vivo analyte level of monitored user 25. Sensor control device 102 can then wirelessly communicate data indicative of the monitored user's analyte levels to reader device 120-1, which, in turn, can wirelessly communicate the data to trusted computer system 180.

According to another aspect of the embodiments, secondary display device 120-2 (which can comprise a smart phone, smart watch, wearable electronic device, or dedicated receiver device) can be configured to receive data indicative of an analyte level of monitored user 25, which is wirelessly transmitted by trusted computer system 180 via network 190 to secondary display device 120-2. Furthermore, secondary display device 120-2 can include a third party analyte monitoring software application, stored in a memory of secondary display device 120-2, where the third party analyte software application can be configured to display analyte information of monitored user 25 to third party 50.

In this manner, third party 50 can receive timely information regarding the analyte information of monitored user 25. In some embodiments, for example, the third party analyte monitoring software application installed on the third party's display device 120-2 can be configured to receive alarms associated with the analyte levels of monitored user 25.

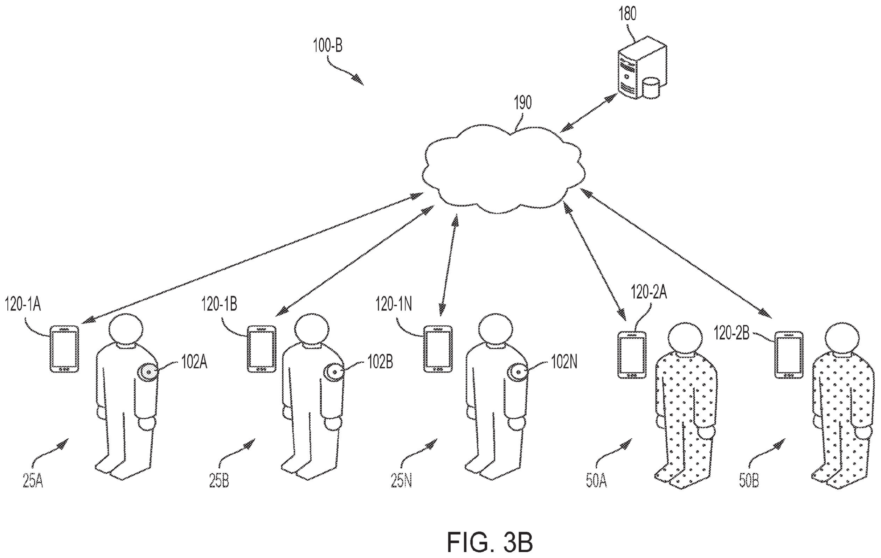

FIG. 3B is a conceptual diagram depicting another example embodiment of a system 100-B capable of communicating analyte data and other related information of one or more monitored users to one or more third parties. According to one aspect of the embodiments, system 100-B shares numerous similarities with systems 100 and 100-A, as described with respect to FIGS. 1 and 3A. In particular, FIG. 3B depicts a system 100-B that is capable of communicating analyte data and other related information from one or more monitored users (25A, 25B, 25N) to one or more third parties (50A and 50B). In some embodiments, for example, system 100-B can comprise one or more sensor control devices, 102A, 102B, 102N (worn by monitored users 25A, 25B, and 25N, respectively), each in wireless communication with a corresponding reader device (e.g., 120-1A, 120-1B, and 120-1N, respectively). Although only one reader device is depicted for each monitored user, those of skill in the art will understand that each monitored user can have multiple reader devices (e.g., smart phones, glucose meters, dedicated receivers), each of which can be configured to receive analyte data from a corresponding sensor control device.

According to another aspect of many of the embodiments, sensor control devices (e.g., 102A, 102B, 102N) can each include communication circuitry configured to wirelessly communicate data with a corresponding reader device (e.g., 120-1A, 120-1B, 120-1N), such as according to a Bluetooth, Bluetooth Low Energy, or an NFC protocol. Furthermore, according to some embodiments, each reader device (e.g., 120-1A, 120-1B, 120-1N) can comprise a smart phone, smart watch, wearable electronic device, dedicated receiver device, or glucose meter, and can be in wireless communication with trusted computer system 180 through network 190. In some embodiments, each reader device (e.g., 120-1A, 120-1B, 120-1N) can include communication circuitry configured to wirelessly communicate data with trusted computer system 180 via an 802.11x communication protocol or a cellular communication protocol.

Referring still to FIG. 3B, system 100-B further comprises one or more secondary display devices (e.g., 120-2A, 120-2B) each belonging to a corresponding one or more third parties (50A, 50B). As previously described, each of the third parties (e.g., 50A, 50B) can comprise a caregiver, parent, spouse, or any individual, party, or entity interested in monitoring the analyte levels of one or more monitored users (e.g., 25A, 25B, 25C). Each of the secondary display devices (e.g., 120-2A, 120-2B) can further include communication circuitry configured to wirelessly communicate data with trusted computer system 180 through network 190 via an 802.11x communication protocol or a cellular communication protocol. According to many embodiments, network 190 can be the Internet.

According to an aspect of the embodiments, each of the sensor control devices (e.g., 102A, 102B, 102N) includes an analyte sensor at least a portion of which is configured to be positioned in the body of a monitored user (e.g., 25A, 25B, 25N). Each of the sensor control devices (e.g., 102A, 102B, 102C) can wirelessly communicate data indicative of an analyte level of a monitored user (e.g., 25A, 25B, 25N) to a corresponding reader device (e.g., 120-1A, 120-1B, 120-1C), which, in turn, can wirelessly communicate the data to trusted computer system 180. According to another aspect of the embodiments, each of the secondary display devices (e.g., 120-2A, 120-2B) can include a third party analyte monitoring application, stored in memory of the secondary display device, configured to be operated by a third party (e.g., 50A, 50B). Furthermore, each of the secondary display devices (e.g., 120-2A, 120-2B), which can comprise a smart phone, smart watch, wearable electronic device, or dedicated receiver device, can be configured to receive data indicative of an analyte level of the monitored user (e.g., 25A, 25B, 25N), which is wirelessly communicated by trusted computer system 180 via network 190 to the third party's display device (e.g., 120-2A, 120-2B).

According to some embodiments, sensor control device 102A, 102B, and 102N can comprise, respectively, a first type of sensor control device, a second type of sensor control device, and a third type of sensor control device. In some embodiments, for example, the first type of sensor control device can be configured to transmit data indicative of the monitored user's analyte levels according to an NFC protocol, and the second type of sensor control device can be configured to transmit data indicative of the monitored user's analyte levels according to a Bluetooth or Bluetooth Low Energy communication protocol. In other embodiments, the first type of sensor control device can be configured to transmit data indicative of the monitored user's analyte levels and "pass through" alarms (i.e., alarm indicators generated according to settings configured by the monitored user). The second type of sensor control device can be configured to transmit data indicative of the monitored user's analyte levels (and, optionally, the monitored user's alarm settings), where the determination of alarm conditions (e.g., whether an analyte threshold has been exceeded) can be performed by either the trusted computer system, the secondary display device, or both. In addition, a third type of sensor control device can comprise an unknown or unsupported sensor control device. As described in further detail below, the third party analyte monitoring software application installed on a third party's display device (120-2A or 120-2B) can be configured to present different types of alarms, alarm settings interfaces, and other features and GUIs based on the type of sensor control device of the monitored user. Additional details regarding different types of alarms for third party analyte monitoring can be found in U.S. Patent Appl. Serial No. 17/478,447, which is hereby incorporated by reference in its entirety for all purposes.

In this manner, each third party (e.g., 50A, 50B) can receive timely information regarding the analyte information of one or more monitored users (e.g., 25A, 25B, 25N). In some embodiments, for example, the third party analyte monitoring software application installed on the third party's display device 120-2A can be configured to receive alarms associated with the analyte levels of monitored users, 25A and 25B. In another embodiment, for example, the third party analyte monitoring software application installed on the third party's display device 120-2B can be configured to receive data indicative of the analyte levels of monitored users, 25A, 25B, and 25N. Similarly, in some embodiments, for example, analyte monitoring software installed on the monitored user's reader device 120-1A can be configured to share data indicative of the analyte levels of monitored user 25A with third party 50A (but not 50B). Those of skill in the art will appreciate that other combinations and permutations are possible and are fully within the scope of the present disclosure.

### Examples of Digital and Graphical User Interfaces for Third Party Analyte Monitoring

Described herein are example embodiments of digital interfaces and GUIs for third party analyte monitoring, as well as systems, methods and devices relating thereto. As an initial matter, it will be understood by those of skill in the art that the GUIs and related methods described herein can comprise instructions stored in a memory of a reader device 120, secondary display device 120-2, local computer system 170, trusted computer system 180, and/or any other device or system that is part of, or in communication with, analyte monitoring systems 100, 100-A, or 100-B. These instructions, when executed by one or more processors of the reader device 120, secondary display device 120-2, local computer system 170, trusted computer system 180, or other device or system of analyte monitoring system 100, 100-A, or 100-B, can cause the one or more processors to perform the method steps and/or output the GUIs described herein. Those of skill in the art will further recognize that the GUIs described herein can be stored as instructions in the memory of a single centralized device or, in the alternative, can be distributed across multiple discrete devices in geographically dispersed locations.

### Example Embodiments of GUIs for Enabling/Disabling Third Party Analyte Monitoring

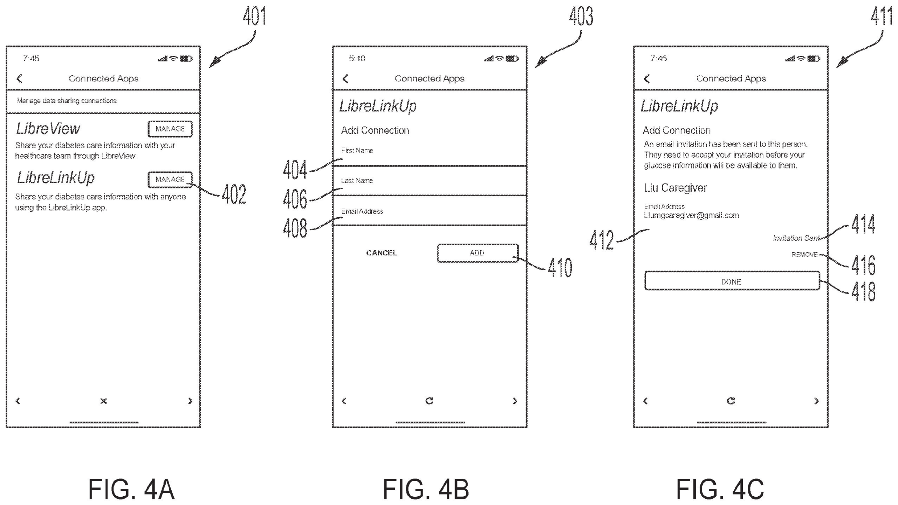

FIGS. 4A to 4I are example embodiments of GUIs for an analyte monitoring software application for enabling, disabling, and configuring third party analyte monitoring. As an initial matter, the GUIs described in this section relate to interfaces for an analyte monitoring software application stored in the memory of a reader device of the monitored user. (By contrast, many of the other GUIs and interfaces described later relate to interfaces for a third party analyte monitoring application stored in the memory of a secondary display device of a third party.)

Referring first to FIG. 4A, GUI 401 depicts an interface for an analyte monitoring software application, wherein the interface allows a monitored user to configure, enable, or disable third party analyte monitoring. According to some embodiments, GUI 401 includes a button 402 that, when pressed, causes another interface to be displayed that allows a monitored user to review and/or configure settings relating to third party analyte monitoring. In some embodiments, GUI 401 can also include other buttons or links that cause other settings interfaces or other features of the analyte monitoring software application to be displayed.

FIG. 4B is another GUI 403 depicting an interface for an analyte monitoring software application, wherein the interface allows the monitored user to enter information about a third party with whom the monitored user wishes to share his or her analyte data. In some embodiments, for example, GUI 403 can include a first name field 404, a last name field 406, and an e-mail address field 408, as well as an "Add" button 410 that generates an invitation to the third party when pressed.

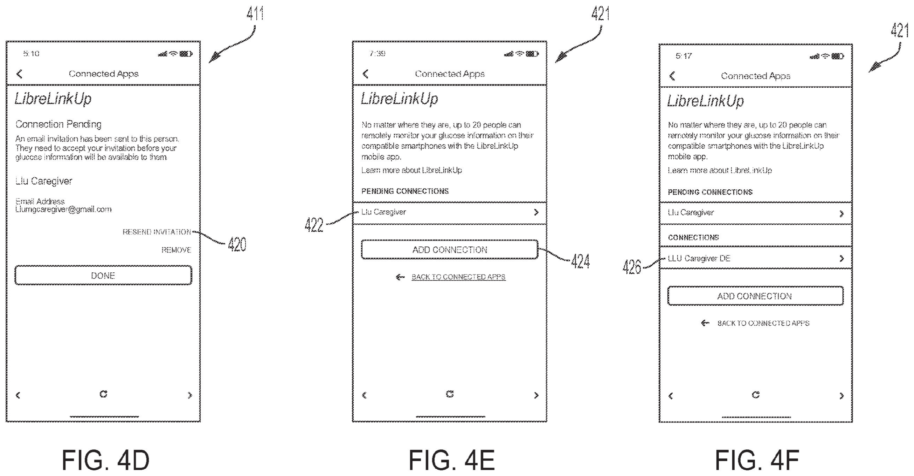

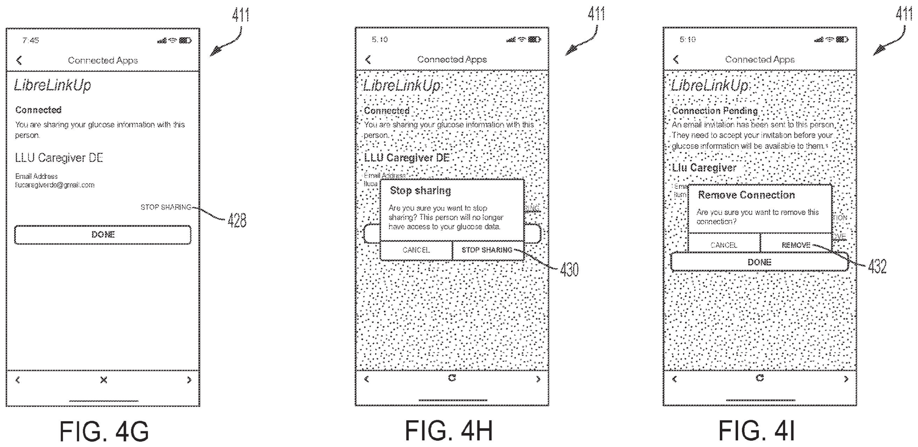

Referring next to FIG. 4C, GUI 411 depicts another interface for an analyte monitoring software application, wherein the interface includes third party label 412 indicating the name and e-mail address of the recently added third party, an invitation status 414 indicating that an invitation to the third party was sent, a remove link 416 to allow a monitored user to disable the sharing of analyte data with a specific third party, and a "Done" button 418 that allows the monitored user to return to a previous screen (e.g., GUIs 401 or 421). FIG. 4D depicts GUI 411 in a different state from FIG. 4C, where a "Resend Invitation" link 420 is provided (instead of the invitation status). In addition, when the remove link 416 is selected, a remove connection modal 432 is displayed, as shown in FIG. 4I.

FIG. 4E is another GUI 421 depicting another interface for an analyte monitoring software application, wherein the interface includes a pending connections section 422 to list any pending connections (e.g., where the third party has not yet responded to an invitation), as well as an "Add Connection" button that, when pressed, returns the monitored user to GUI 403. FIG. 4F depicts GUI 421 in a different state from FIG. 4E, wherein the interface includes a pending connections section (as described above), as well as a connections section 426 to list all established connections (e.g., where the third party has accepted the invitation to share the monitored user's analyte data).

Referring next to FIG. 4G, GUI 411 is depicted in a different state from the interface shown in FIGS. 4C and 4D. In particular, FIG. 4G depicts GUI 411 after the third party has accepted the invitation and a connection has been established. In addition, in some embodiments, GUI 411 also includes a Stop Sharing link 428 that, when pressed, displays a Stop Sharing modal 430, as shown in FIG. 4H. According to one aspect of the embodiments, the Stop Sharing modal 430 allows the monitored user to discontinue sharing analyte data (and other related information) with the third party.

Although each of the individual graphical elements (e.g., modals, buttons, and links) is described with respect to specific interfaces, those of skill in the art will appreciate that each and any of these elements can be re-arranged in various configurations within a single interface, or combined with other elements from different interfaces, to achieve the same functionality and/or result, and that such other combinations and/or arrangements of graphical elements and interfaces are fully within the scope of the present disclosure.

### Example Embodiments of GUIs for Setup of a Third Party Analyte Monitoring Application

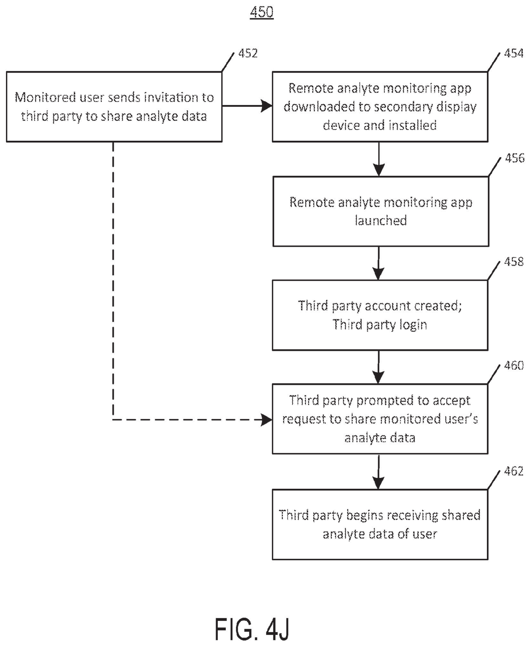

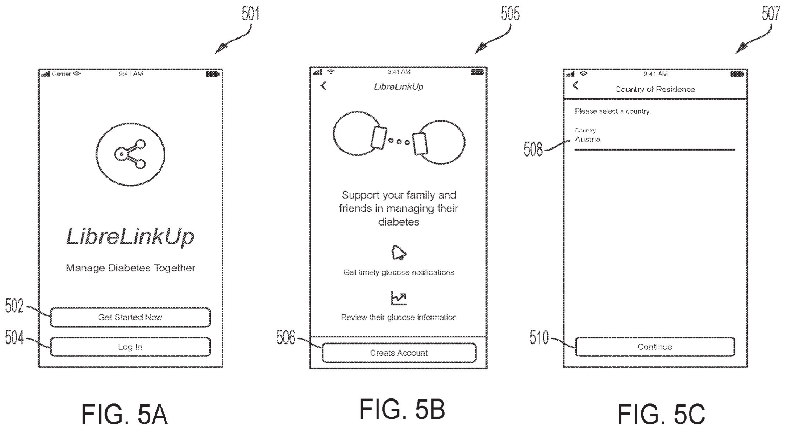

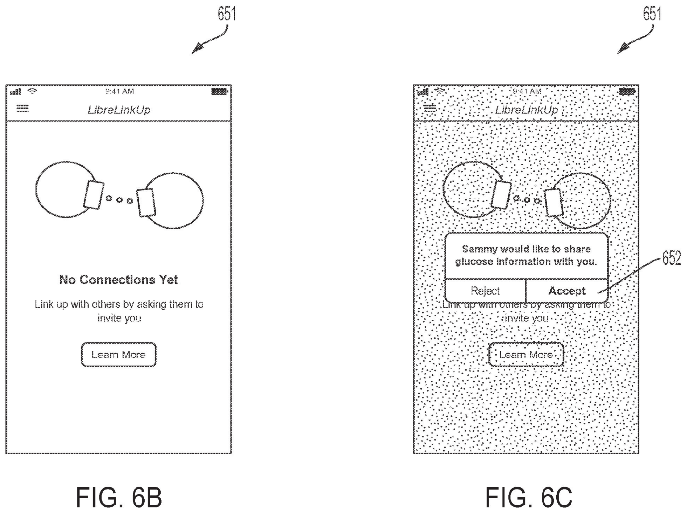

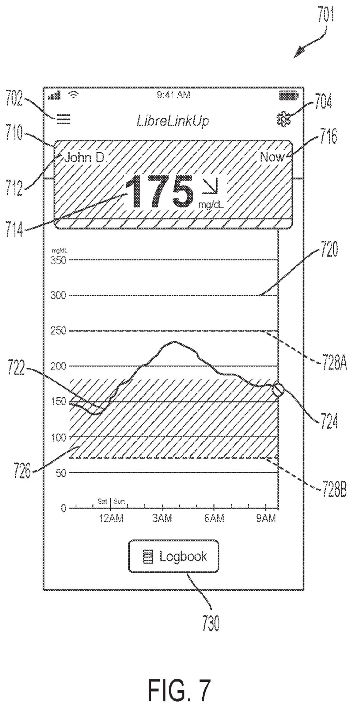

FIG. 4J is a flow diagram depicting an example embodiment of a method 450 for setting up a third party analyte monitoring application (also referred to as a third party analyte software monitoring application or third party analyte monitoring app) to allow for third party analyte monitoring by, for example, a caregiver, parent, or spouse. At Step 452, a monitored user sends an invitation to a third party to share analyte data and other information. According to some embodiments, the monitored user can send the invitation via an analyte monitoring software application residing on the monitored user's reader device, as previously described with respect to FIGS. 4A to 4H. In other embodiments, the monitored user can send an invitation to a third party through a web-based interface (e.g., through a web browser) that is separate from the analyte monitoring application. In many embodiments, the invitation can comprise an e-mail received by the third party containing links to download and install the third party analyte monitoring application. At Step 454, if the third party has not already done so, the third party analyte monitoring application is downloaded to the third party's display device (also referred to as the secondary display device) and installed. Subsequently, at Step 456, the third party analyte monitoring application is launched on the third party's display device. At Step 458, the third party analyte monitoring application prompts the third party to create a user account or, if the third party already has a user account, prompts the third party to login to a trusted computer system (e.g., a cloud-based server) through the third party analyte monitoring application. (See, e.g., FIGS. 5A to 5Q.) After the third party has successfully logged in, then, at Step 460, the third party receives a notification, through the third party analyte monitoring application, that the monitored user wishes to share his or her analyte data, and the third party is prompted to accept the request. (See, e.g., FIG. 6C.) If the third party accepts the request, then, at Step 462, the third party analyte monitoring application begins to receive and display analyte levels of the monitored user and other related information on the third party's display device. (See, e.g., FIGS. 7, 8A to 8J, and 9A to 9C.)

Example embodiments of GUIs for the setup of a third party analyte monitoring application will now be described. It will be understood by those of skill in the art that the interfaces described in this section can be used in combination with any of the other embodiments described herein, including, but not limited to, method 450 and the interfaces described with respect to FIGS. 4A to 4I.

Referring first to FIG. 5A, GUI 501 depicts an initial interface displayed when the third party analyte monitoring application is launched. According to one aspect of the embodiments, GUI 501 includes a "Get Started Now" button 502 for a first-time third party user and a "Log In" button 504 if the third party already has a user account.

Turning next to FIG. 5B, GUI 505 depicts an introductory interface displayed when the third party selects the "Get Started Now" button 502 in GUI 501. As shown in FIG. 5B, GUI 505 can include informational text regarding the third party analyte monitoring application and a "Create Account" button 506.

Referring next to FIG. 5C, GUI 507 depicts a region setting interface displayed when the third party selects the "Create Account" button 506 in GUI 505. As shown in FIG. 5C, GUI 507 can include a pulldown menu 508, dial, or free text field that allows the third party to input a country or region. According to some embodiments, certain features of the third party analyte monitoring application can be enabled or disabled based on the country or region inputted by the third party. In other embodiments, the unit of measure ("UOM") displayed on various interfaces can also depend on the country or region inputted by the third party.

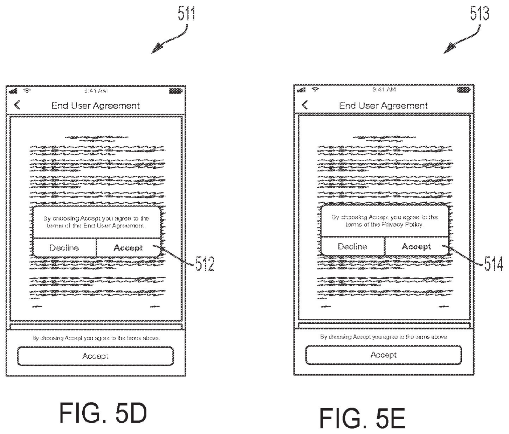

After the country or region is inputted using menu 508 and the "Continue" button 510 is selected, the third party analyte monitoring application displays, respectively, an End User License Agreement ("EULA") 511 and Privacy Policy 513, each of which includes a modal 512, 514 that prompts the third party to accept the terms of each agreement. These are depicted in FIGS. 5D and 5E. In some embodiments, the third party analyte monitoring application cannot progress beyond these interfaces if the third party does not accept the EULA or terms of the Privacy Policy.

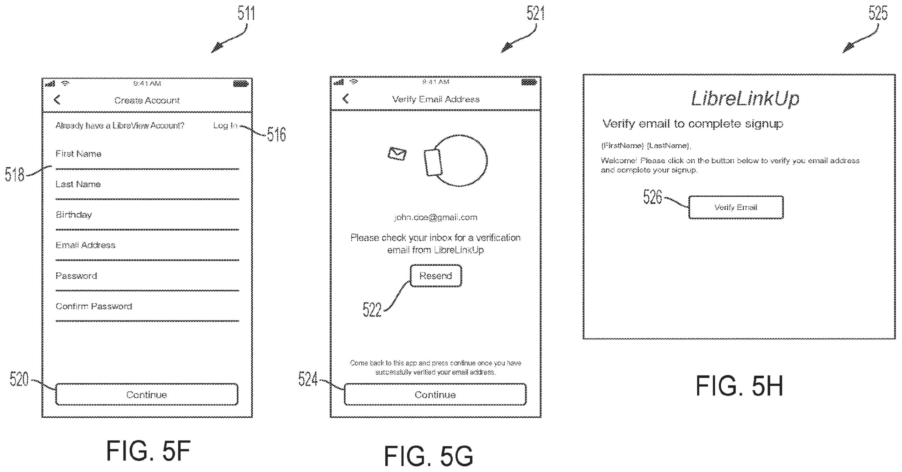

Turning next to FIG. 5F, GUI 511 depicts an interface of the third party analyte monitoring application that allows the third party to create a user account. According to one aspect of some embodiments, GUI 511 includes a plurality of fields 518 with which the third party can input information needed to create a user account, such as, e.g., first name, last name, birthday, e-mail address, password, and password confirmation. After the requested information is inputted by the third party into the plurality of fields 518 and the "Continue" button 520 is selected, the third party is then prompted to verify the inputted e-mail address, as shown in GUI 521 of FIG. 5G. Furthermore, GUI 521 includes a "Resend" link, in case the third party inadvertently deletes or loses the original verification e-mail. (FIG. 5H depicts an example embodiment of an e-mail message containing a verification link 526 to verify the third party's new account. Although FIG. 5H shows an e-mail message addressed to a user by a "FirstName" field followed by a "LastName" field, those of skill in the art will understand that the e-mail message can be addressed to the user in other ways. According to some embodiments, for example, the user can be addressed by a "LastName" followed by a "FirstName" if the user is in a predetermined country, region, or market.)

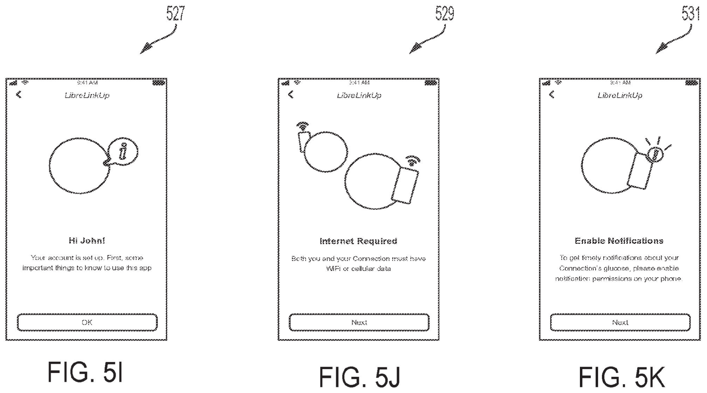

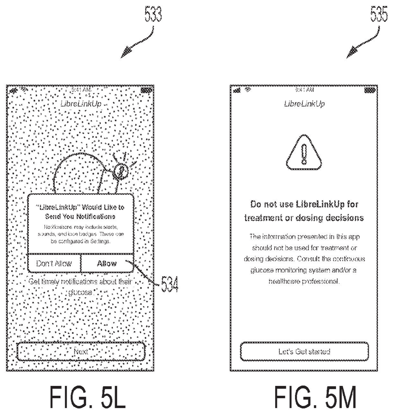

According to another aspect of the embodiments, once the verification process is complete, and the third party selects the "Continue" button 524 of GUI 521 (FIG. 5G), a series of informational and configuration interfaces, FIGS. 5I to 5M, are displayed to the third party. In some embodiments, for example, GUI 527 (FIG. 5I) is first displayed after account creation, wherein the interface comprises a confirmation message that the third party's account has been successfully created. Similarly, GUI 529 (FIG. 5J) can display a message that Internet connectivity is required (e.g., either via a Wi-Fi or cellular connection) in order for the third party analyte monitoring application to function properly. In addition, GUI 531 (FIG. 5K) displays instructions to the third party to enable notification permissions through the display device's operating system. Subsequently, a modal 534 can be presented to the third party, as shown in GUI 533 of FIG. 5L, so that the requisite notification permissions can be enabled by the third party. In some embodiments, the third party analyte monitoring application can be configured such that the application is either inoperable or partially operable until the notification permissions have been properly enabled. Finally, as shown in FIG. 5M, a GUI 535 can display a warning that the third party analyte monitoring application is not to be used for treatment or dosing decisions.

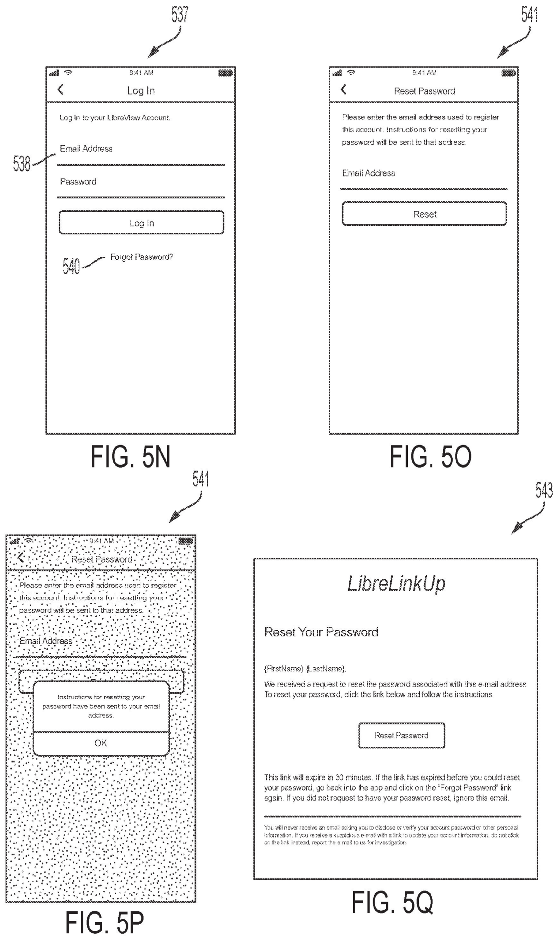

Referring back to FIGS. 5A and 5F, if the third party already has an account, then a "Log In" button 504 or link 516 can be selected, which can cause display of a login GUI 537, as shown in FIG. 5N. According to one aspect of the embodiments, the third party can enter his or her e-mail address and password into the respective login fields 538 to login to his or her user account. Alternatively, if the third party has forgotten his or her password, the "Forget Password" link 540 can be selected, which will cause a series of Password Reset interfaces to be displayed. *See, e.g.,* GUIs 541 and 543 of FIGS. 5O, 5P, and 5Q.

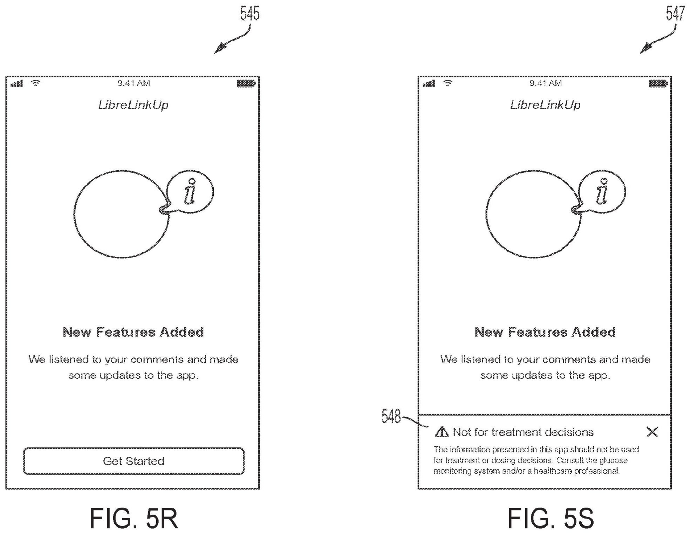

According to another aspect of the embodiments, if the third party has successfully logged in, a "New Features" interface 545, as shown in FIG. 5R, can be displayed. In some embodiments, the "New Features" interface 545 can be displayed only if the user has not logged in since the last update to the third party analyte monitoring application. In other embodiments, the "New Features" interface 545 can be displayed according to a pre-defined schedule. In some embodiments, a "New Features" interface 547 can include a safety banner notification 548 to further emphasize that the third party analyte monitoring application is not for treatment decisions. In some embodiments, the safety banner notification 548 can be configured such that it will auto-dismiss after a predetermined amount of time (e.g., 5 seconds, 15 seconds, 30 seconds, etc.). In other embodiments, the safety banner notification 548 can be configured to persist on the interface 547 until the third party manually dismisses the notification 548 to ensure that the third party has had sufficient time to view and read the message. Although the safety banner notification 548 is shown with respect to the "New Features" interface, those of skill in the art will understand that the same or similar safety banner notification 548 can be temporarily overlaid onto any of the interfaces described herein based upon one or more different conditions such as, for example, a login, when the third party analyte monitoring application is brought into the foreground, or a specific predetermined interface being displayed. Furthermore, although the banner notification 548 depicted in FIG. 5S relates to treatment decisions, those of skill in the art will further understand that other urgent or important messages can be displayed and are fully within the scope of the present disclosure.

### Example Embodiments of Connection Interfaces for a Third Party Analyte Monitoring Application

Example embodiments of connection interfaces for a third party analyte monitoring application will now be described. As an initial matter, a "connection interface" refers to an interface for presenting data indicative of the analyte levels of one or more monitored users. It will also be understood by those of skill in the art that the interfaces described herein can comprise instructions stored in memory of a secondary display device (e.g., smart phone, smart watch, wearable electronic device, dedicated receiver, etc.) belonging to a third party.

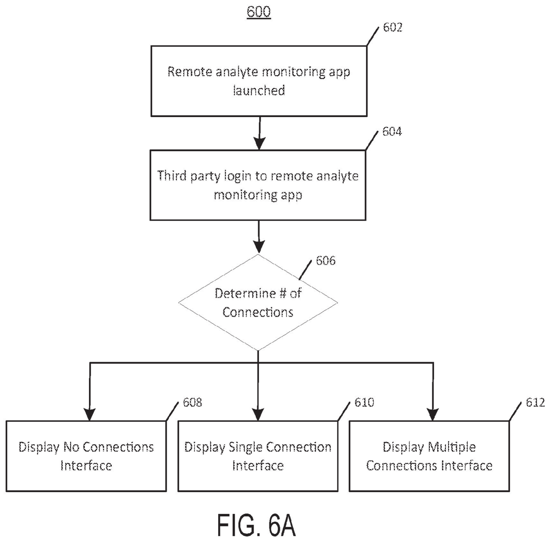

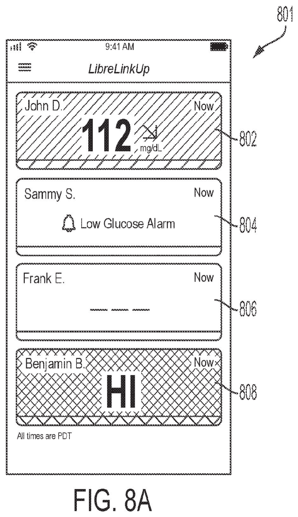

FIG. 6A is a flow diagram depicting an example embodiment of a method 600 for displaying a connection interface in a third party analyte monitoring application. At Step 602, the third party analyte monitoring application is launched on a secondary display device belonging to a third party. As described earlier, the secondary display device can comprise a smart phone, smart watch, wearable electronic device, or a dedicated receiver device. At Step 604, the third party logs in with his or her account and password via the third party analyte monitoring application. In some embodiments, the third party analyte monitoring application passes along the third party's credentials to a cloud-based server for authentication purposes. In other embodiments, the third party's user account and password can be locally authenticated by the third party analyte monitoring application itself, or by the secondary display device. At Step 606, a determination is made as to how many connections are active for the third party based on the third party's user account. If the third party has not yet established any connections, then, at Step 608, a no connections interface is displayed. (See, e.g., FIG. 6B.) If the third party has one connection, then a single connection interface is displayed at Step 610. (See, e.g., FIG. 7.) If the third party has multiple connections, then a multiple connections interface is displayed on the secondary display device at Step 612. (See, e.g., FIG. 8A.)

Referring to FIG. 6B, GUI 651 depicts an example embodiment of a no connections interface to be displayed by the third party analyte monitoring application if the third party has no active connections. In many embodiments, GUI 651 can include informational text indicating that there are "No Connections Yet," and can further include a "Learn More" link to provide additional instructions and/or information with respect to adding connections. FIG. 6C depicts GUI 651 in another state where a monitored user has invited the third party to share analyte data, in which case a modal 652 is displayed to the third party. According to some embodiments, the third party can either accept or reject the request to share via the respective buttons of modal 652. In other embodiments, modal 652 can be informational only, and a connection can be automatically established.

Referring next to FIG. 7, GUI 701 depicts an example embodiment of a single connection interface to be displayed by the third party analyte monitoring application. According to one aspect of the embodiments, GUI 701 can comprise a menu icon 702, a settings icon 704, a profile card section 710, an analyte graph section 720, and a logbook button 730. In many embodiments, the profile card section 710 can further comprise a connection name 712 (e.g., the name of the monitored user, which can be displayed as: a first name followed by an initial of a last name (as shown in FIG. 7); in full, as a first name followed by a last name; or, alternatively, in full, as a last name followed by a first name for certain predetermined countries or markets), a time stamp 716, and a current analyte level value 714. In some embodiments, the current analyte level value 714 can comprise a numeric value (which can be displayed in a unit of measure determined by the third party's region or country), and a directional trend arrow to indicate whether the monitored user's analyte level is rising or falling. In addition, the profile card section 710 can have a background color to indicate whether the monitored user's analyte level is above, below, or within a target analyte range. For example, if the monitored user's analyte level is below a target range, the background color of the profile card section 710 can be red. If the monitored user's analyte level is within a target range, the background color of the profile card section 710 can be green. According to some embodiments, the target range can be a predetermined analyte level range configured by the monitored user. In other embodiments, the target range can be configured by the third party through the third party analyte monitoring application.

Referring still to FIG. 7, an analyte graph section 720 is located adjacent to the profile card section 710, and can include an analyte trend line 722 to reflect the monitored user's analyte levels over a predetermined amount of time. For example, as shown in FIG. 7, the x-axis of the analyte graph section 720 can comprise units of time (e.g., in five minute increments, fifteen-minute increments, etc.) covering a twelve-hour period, whereas the y-axis can comprise a measured analyte concentration of the monitored user. Those of skill in the art will further recognize that other predetermined periods of time (e.g., four hours, twenty-four hours, forty-eight hours, etc.) can be reflected on the x-axis, and are fully within the scope of the present disclosure.

Analyte graph section 720 can further include: a shaded area to indicate the monitored user's target range, as indicated by shaded area 726, as well as one or more alarm thresholds, depicted in FIG. 7 as dashed lines 728A (high glucose threshold) and 728B (low glucose threshold). In some embodiments, the alarm thresholds can be configured by the monitored user. In other embodiments, the alarm thresholds can be configured by the third party through the third party analyte monitoring application. In still other embodiments, the analyte graph section 720 can have no alarm thresholds (e.g., if the third party or monitored user has disabled alarms, or if the monitored user's sensor control device does not support alarms).

According to another aspect of many embodiments, the connection interface can be real-time, or near real-time, and interactive. In some embodiments, for example, the information reflected in the profile card section 710 (e.g., current analyte level value 714) and analyte graph section 720 (e.g., analyte trend line 722) can be automatically updated and/or refreshed at a predetermined frequency (e.g., every thirty seconds, every minute, every five minutes, etc.). In some embodiments, the current analyte level value 714 and analyte trend line 722 can be updated when the connection interface is rendered in the foreground of the secondary display device. In still other embodiments, the current profile card section 710 and analyte graph section 720 can be updated in response to a predetermined input by the third party, such as when the third party pulls the screen down with a finger, or by some other predetermined gesture.

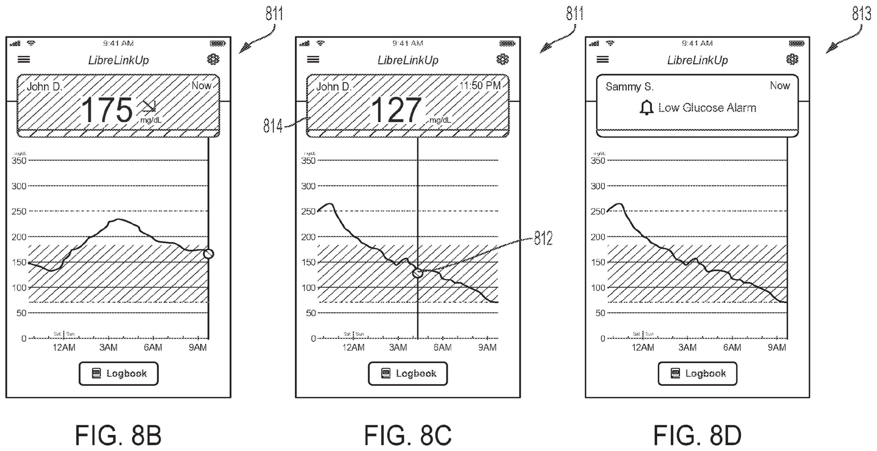

In addition, according to another aspect of the embodiments, profile card section 710 can be configured to display historical analyte data based on the third party's interaction with analyte graph section 720. In some embodiments, for example, when the third party interacts with a point on analyte trend line 722 (e.g., by touching a point on the analyte trend line 722), profile card section 710 can be updated to display historical analyte level values based on the location of the point along analyte trend line 722. Likewise, time stamp 716 and background color of the profile card section 710 can also be updated to reflect the time stamp of the historical analyte level value being displayed, and whether the historical analyte level value was within or outside of a target analyte range, in accordance with the point or portion of the analyte trend line 722 being interacted with. See, e.g., FIG. 8C.

As shown in FIG. 7, the point or portion of the analyte trend line 722 being displayed in profile card section 710 can be indicated by a colored circle 724 on analyte trend line 722. In some embodiments, a third party can touch colored circle 724 and drag it along analyte trend line 722, and the information displayed in the profile card section 710 can be immediately updated with historical analyte level data based on the point or portion of the analyte trend line 722 being touched by the third party. *See, e.g.,* FIG. 8C. According to one aspect of some embodiments, the connection interface can also be configured to return the selected point to the current time (also referred to as current glucose level) in response to the third party tapping on profile card section 710. In some embodiments, the connection interface can be configured to return the selected point to the current time/current glucose level in response to the third party tapping on a right edge portion of analyte graph section 720. In some embodiments, the connection interface can be configured to return the selected point to the current time/current glucose level in response to the third party tapping on any portion of analyte graph section 720 in combination with a dragging gesture to the right. Those of skill in the art will recognize that any of the aforementioned gestures can be implemented alone or in combination.

FIGS. 8A to 8J depict example embodiments of connection interfaces to be displayed by a third party analyte monitoring application when the third party has multiple connections. Referring first to FIG. 8A, GUI 801 depicts a multiple connections interface comprising a plurality of profile cards 802, 804, 806, and 808. Generally, each of the profile cards of GUI 801 is similar to profile card section 710, as described with respect to FIG. 7. For example, profile card 802 comprises a connection name, a time stamp, and a current analyte level value (including a numeric value and a directional trend arrow). As described earlier, each connection name can be displayed as: a first name followed by an initial of a last name (as shown in FIG. 8A); in full, as a first name followed by a last name; or, alternatively, in full, as a last name followed by a first name for certain predetermined countries or markets. In addition, profile card 802 can have a background color to indicate whether the monitored user's analyte level is within or outside of a target analyte range. According to another aspect of the embodiments, if the third party selects profile card 802 (e.g., by pressing a corresponding area of the touchscreen), a corresponding individual connection interface 811 is then displayed, as shown in FIGS. 8B and 8C. As described earlier, the third party can then interact with GUI 811 by dragging the colored circle 812 along the analyte trend line and causing the profile card section 814 to display a corresponding historical analyte level value and corresponding time stamp associated with the point of the analyte trend line where colored circle 812 is located.

Referring back to FIG. 8A, profile card 804 also comprises a connection name and a time stamp. However, instead of a current analyte level value, profile card 804 displays a low glucose alarm text and icon to indicate that a low glucose threshold condition has been triggered. According to some embodiments, if the third party selects profile card 804, a corresponding individual connection interface 813, as shown in FIG. 8D, is then displayed. In some embodiments, however, GUI 813 maintains the "Low Glucose Alarm" and icon in the profile card section, and the analyte trend line is static and cannot be interacted with. In other embodiments, the analyte trend line in interface 813 can continue to update and, furthermore, can be interacted with by the third party, but the trend line may display a gap.

Referring back to FIG. 8A, profile card 806 also comprises a connection name and a time stamp. However, instead of a current analyte level value, profile card 806 displays three dashes to indicate that no recent data has been received. This can be due to a signal loss condition such as, for example, the monitored user's smart phone malfunctioning or going outside a wireless communication range with the monitored user's on-body sensor control device. Additional details regarding signal loss conditions can be found in U.S. Patent Appl. Serial No. 17/478,447, which is hereby incorporated by reference in its entirety for all purposes.

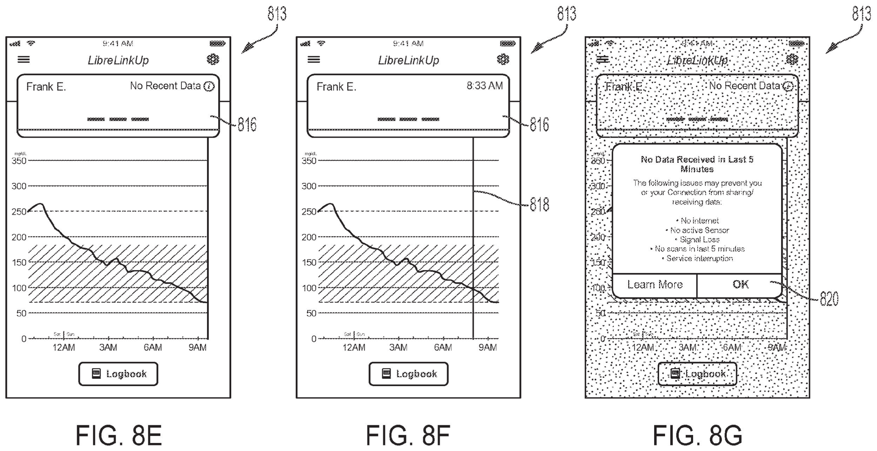

According to another aspect of some embodiments, if the third party selects profile card 806, a corresponding individual connection interface 813, as shown in FIGS. 8E, 8F, and 8G, is then displayed. As described earlier, the third party can then interact with GUI 813 by touching the analyte trend line (or an adjacent area), causing the profile card section 816 to display a corresponding historical analyte level value (if any) and a corresponding time stamp associated with the point of the analyte trend line being interacted with by the third party. For example, FIG. 8F shows that, at the selected point 818, there is no historical analyte data, as indicated by the three dashes, at the corresponding time of 8:33 A.M. Referring back to FIG. 8E, profile card section 816 also indicates that there is "No Recent Data" along with an informational icon. In some embodiments, pressing the informational icon will further cause display of an information modal 820, as shown in FIG. 8G, where the modal can provide additional information regarding potential causes for the "No Recent Data" condition.

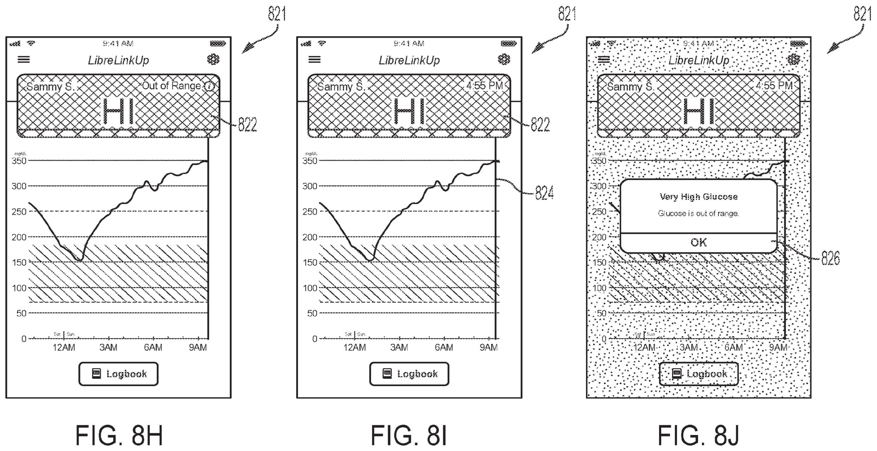

Referring back to FIG. 8A, profile card 808 also comprises a connection name and a time stamp. However, instead of a current analyte level value, profile card 808 displays the text, "HI" to indicate that the monitored user's analyte level is above a reportable analyte level upper limit. (Similarly, a textual "LO" indicator can indicate that the monitored user's analyte level is below a reportable analyte level lower limit.) As seen in FIG. 8A, profile card 808 can comprise an orange background color to indicate that the analyte level value is above a target analyte range. If the third party selects profile card 808, a corresponding individual connection interface 821, as shown in FIGS. 8H, 8I, and 8J, is then displayed. As described earlier, the third party can then interact with GUI 821 by touching the analyte trend line (or an adjacent area), causing the profile card section 822 to display a corresponding historical analyte level value (if any) and a corresponding time stamp associated with the point of the analyte trend line being interacted with by the third party. For example, FIG. 8I shows that, at the selected point 824, the monitored user's analyte level is above the reportable upper limit at a corresponding time of 4:55 P.M. Referring back to FIG. 8H, profile card section 822 also includes a message that the monitored user's analyte level is "Out of Range," along with an informational icon. In some embodiments, pressing the informational icon can cause display of modal 826, as shown in FIG. 8J, where the modal can provide additional information regarding the out-of-range glucose condition.

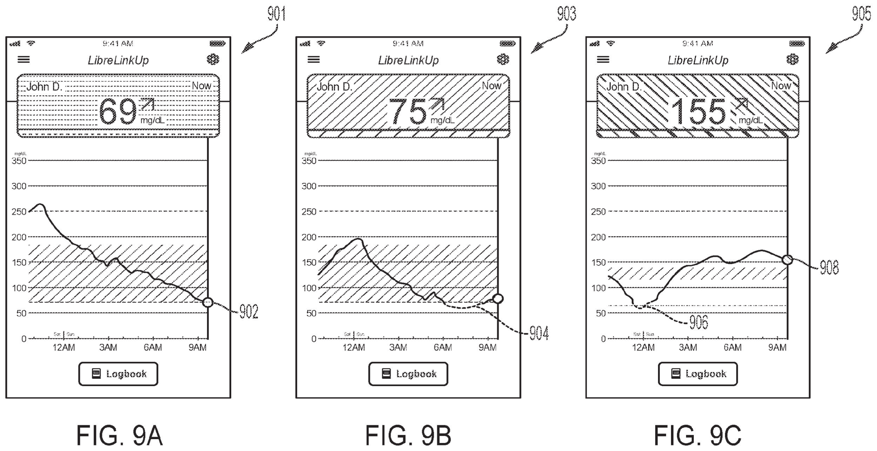

FIGS. 9A to 9E are additional example embodiments of connection interfaces 901, 903, 905, 909, and 911. FIG. 9A is connection interface 901, which includes a colored dot 902 having a red color to indicate that a selected point is below the target range. According to some embodiments, colored dot 902 can be displayed in a predetermined color based on whether the location of the dot along the analyte trend line reflects a value that is above, below, or within a predetermined target range. For example, in FIG. 9B, the colored dot is green, indicating that the selected point is within a target analyte range. In FIG. 9C, the colored dot 908 is orange, indicating that the selected point is above a target analyte range.

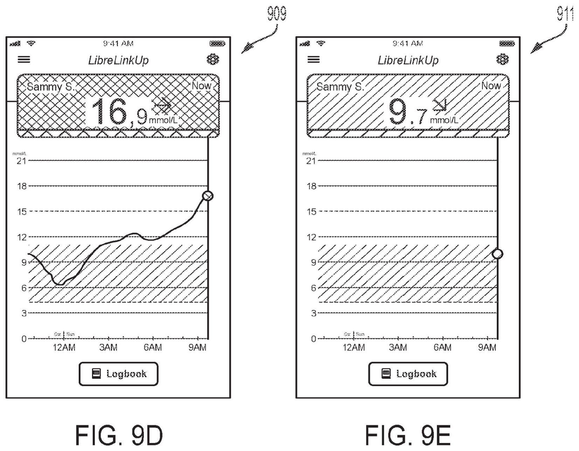

Referring to FIG. 9B, connection GUI 903 includes an analyte trend line, a portion 904 of which is displayed in a red color to indicate that certain historical analyte level values are below a fixed low glucose threshold. According to some embodiments, the fixed low glucose threshold can be a threshold value (e.g., below 70 mg/dL) that is independent of the target range and/or alarm thresholds, as illustrated in FIG. 9C where the red portion 906 of the analyte trend line is independent of the low glucose alarm threshold and the target analyte range. Referring next to FIGS. 9D and 9E, connection GUIs 909 and 911 depict interfaces utilizing a different unit of measure (mmol/L) relative to FIGS. 9A to 9C (mg/dL). The different units of measure are indicated in the current analyte value of the profile card section, as well as along the y-axis of the analyte graph section. In some embodiments, the units of measure can be a configurable setting within the third party analyte monitoring application. In other embodiments, the units of measure can be determined based on the build of the third party analyte monitoring application, or based on the selection of the region and/or country during setup.

According to another aspect of the embodiments, analyte data displayed in the connection interfaces can reflect merged analyte data collected from multiple reader devices utilized by a monitored user. In some embodiments, for example, a monitored user may collect analyte data from the sensor control device using multiple reader devices, such as, for example, a smart phone, a dedicated receiver device, and/or a glucose meter. Each of the reader devices of the monitored user can transmit collected analyte data to the trusted computer system 180 (e.g., a cloud-based server). Thereafter, in many embodiments, the collected analyte data can be merged by trusted computer system 180, e.g., to resolve discrepancies or remove duplicate records, before the data is transmitted to the third party's secondary display device. In other embodiments, the collected analyte data can be merged by the third party analyte monitoring application on the third party's secondary display device. Further details regarding methods by which to merge analyte data collected by multiple reader devices can be found in U.S. Publ. No. 2021/0378601A1, which is hereby incorporated by reference in its entirety for all purposes.

Those of skill in the art will recognize that any one or more of these methods or conditions for updating or interacting with displayed analyte information can be implemented either alone or in combination with each other, and furthermore, can depend on the monitored user's configured settings, the third party's configured settings, or the type of sensor control device worn by the monitored user.

### Example Embodiments of Logbook Interfaces for Third Party Analyte Monitoring Applications

Example embodiments of logbook interfaces for a third party analyte monitoring application will now be described. As an initial matter, it will be understood by those of skill in the art that the interfaces described herein can comprise instructions stored in memory of a secondary display device (e.g., smart phone, smart watch, wearable electronic device, dedicated receiver, etc.) belonging to a third party.

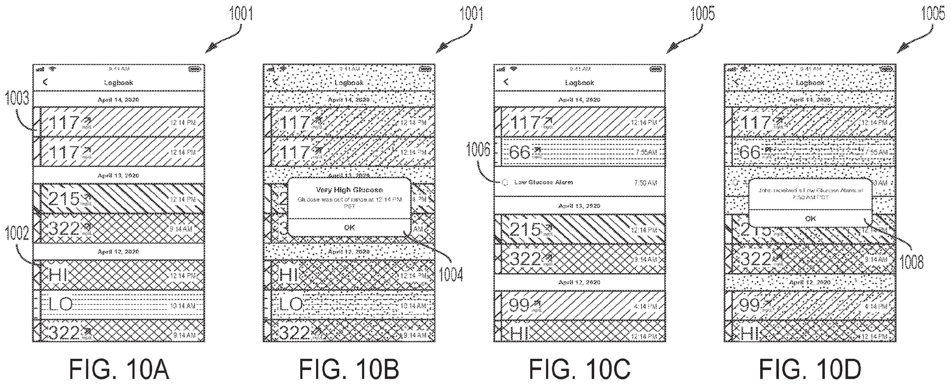

As described earlier with respect to FIG. 7, each connection interface can include a logbook button 730. According to one aspect of the embodiments, if logbook button 730 is selected, a logbook interface is then displayed in the third party analyte monitoring application, as shown in FIGS. 10A to 10H. Each logbook interface can comprise a plurality of logbook entries for a predetermined period of time (e.g., last two weeks, last month, last six months). According to another aspect of the embodiments, the logbook interface can include logbook entries from different types of sensor control devices. In some embodiments, for example, a logbook entry can be automatically generated when a monitored user's sensor control device is scanned by the monitored user's reader device. In other embodiments, a logbook entry can be automatically generated when an alarm is triggered and/or presented on a monitored user's reader device. In still other embodiments, a logbook entry can be automatically generated when an alarm is triggered and/or presented on the third party's secondary display device. In other embodiments, a logbook entry can be manually inputted by the monitored user in an analyte monitoring application of the monitored user's reader device.

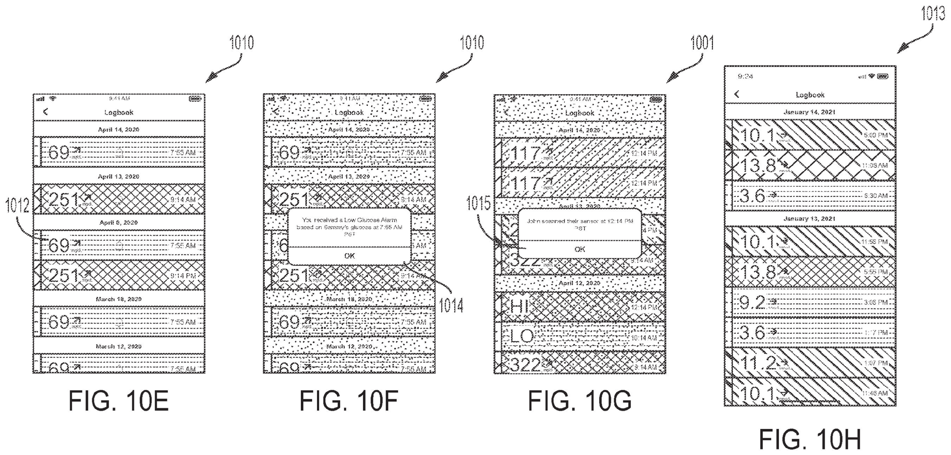

FIG. 10A depicts an example embodiment of a logbook GUI 1001 for use in a third party analyte monitoring application. Logbook GUI 1001 comprises a plurality of logbook entries grouped by date, wherein each logbook entry can comprise an analyte level value, a directional trend arrow, a background color, and a time stamp. For example, logbook GUI includes logbook entry 1003, which indicates an analyte reading at 12:14 pm by the monitored user, where the analyte level is 117 mg/dL, the analyte level is rising (e.g., upward trend arrow), and that the analyte level is within a target analyte range (e.g., green background). According to another aspect of the embodiment, if log entry 1003 is selected, an informational modal 1015, as shown in FIG. 10G, is displayed providing additional information about the log entry. In addition, logbook GUI 1001 can further include logbook entries that indicate when the monitored user's analyte level is above a reportable analyte level upper limit. For example, logbook entry 1002 displays a textual "HI" indicator to indicate that the monitored user's analyte level is above a reportable analyte level upper limit. (Similarly, a textual "LO" indicator can indicate that the monitored user's analyte level is below a reportable analyte level lower limit.) Furthermore, according to the depicted embodiments, selecting log entry 1002 can cause the display of an informational modal 1004, as shown in FIG. 10B, where the modal can provide additional information regarding the out-of-range condition.

FIG. 10C depicts another example embodiment of a logbook GUI 1005 for use in a third party analyte monitoring application. FIG. 10C depicts a logbook interface that is similar to the logbook GUI 1001 described with respect to FIGS. 10A and 10B, except that GUI 1005 further includes logbook entry 1006 reflecting a low glucose alarm. As described earlier, logbook entry 1006 can be generated automatically in response to a low glucose alarm or a high glucose alarm being triggered and/or presented on the monitored user's reader device. Furthermore, according to the depicted embodiments, selecting log entry 1005 can cause the display of an information modal 1008, as shown in FIG. 10D, where the modal can provide additional information about the low glucose alarm (e.g., time of alarm).

FIG. 10E depicts another example embodiment of a logbook GUI 1010 for use in a third party analyte monitoring application. FIG. 10E depicts a logbook interface that is similar to logbook GUIs 1001 and 1005, except that all logbook entries shown in GUI 1010 are automatically generated in response to alarms triggered and/or presented on the third party's secondary display device. For example, log entry 1012 comprises an analyte level value (e.g., 69 mg/dL), a directional trend arrow, a background color, an alarm icon, and a time stamp. Furthermore, according to the depicted embodiments, selecting log entry 1010 can cause the display of an information modal 1014, as shown in FIG. 10F, where the modal can provide additional information about the low glucose alarm (e.g., time of alarm).

Additionally, as with the connection interfaces, the analyte data displayed in the logbook interfaces can reflect merged analyte data collected from multiple reader devices utilized by a monitored user. In some embodiments, for example, a monitored user may collect analyte data from the on-body sensor control device using multiple reader devices, such as, for example, a smart phone, a dedicated receiver device, and/or a glucose meter. Each of the reader devices of the monitored user can transmit the collected analyte data to the trusted computer system 180 (e.g., a cloud-based server). Thereafter, in many embodiments, the collected analyte data can be merged by trusted computer system 180, e.g., to resolve discrepancies or remove duplicate records, before it is transmitted to the third party's secondary display device. In other embodiments, the collected analyte data can be merged by the third party analyte monitoring application on the third party's secondary display device. Further details regarding methods by which to merge analyte data collected by multiple reader devices can be found in U.S. Publ. No. 2021/0378601A1, which is hereby incorporated by reference in its entirety for all purposes.

Those of skill in the art will also recognize that any of the connection interfaces depicted and described herein, including the logbook interfaces, can be configured to display analyte level values in different units of measure. For example, logbook GUI 1001 (FIG. 10A) depicts analyte level values in units of "mg/dL," whereas logbook GUI 1013 (FIG. 10H) depicts analyte level values in units of "mmol/L." In addition, those of skill in the art will appreciate that the different types of logbook interfaces described herein can be implemented either alone or in combination with each other, and furthermore, can depend on the monitored user's configured settings, the third party's configured settings, or the type of sensor control device worn by the monitored user.

### Example Embodiments of Alarm Notification Settings Interfaces for Third Party Analyte Monitoring Applications

Example embodiments of alarm notification settings interfaces for a third party analyte monitoring application will now be described. As an initial matter, it will be understood by those of skill in the art that the interfaces described herein can comprise instructions stored in memory of a secondary display device (e.g., smart phone, smart watch, wearable electronic device, dedicated receiver, etc.) belonging to a third party.

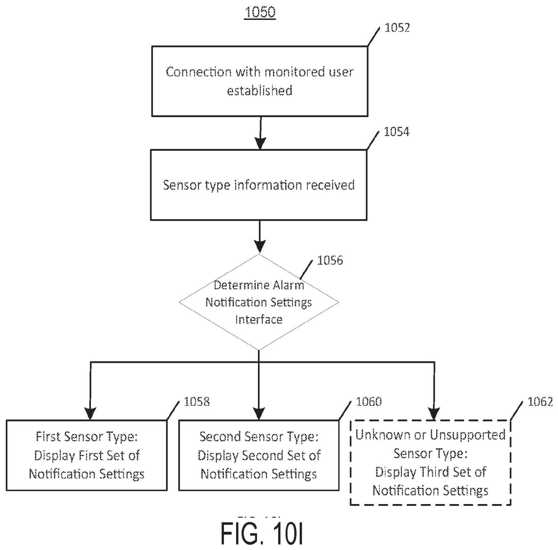

According to one aspect of the embodiments, the third party analyte monitoring application can be configured to present one or more alarm notification settings interfaces based on the type of sensor control device worn by the monitored user. FIG. 10I is a flow diagram depicting an example embodiment of a method 1050 for displaying alarm notification settings interfaces for a third party analyte monitoring application. As an initial matter, prior to Step 1052, method 1050 can require that the third party first install the third party analyte monitoring application on the secondary display device (e.g., a smart phone, smart watch, wearable electronic device, or a dedicated receiver device), and create a user account, as described earlier with respect to FIGS. 5A to 5M. At Step 1052, a connection with a monitored user is established via the third party analyte monitoring application on the third party's secondary display device. The process for establishing the connection can utilize the methods and interfaces described with respect to FIGS. 4A to 4J and 6B to 6C. At Step 1054, sensor type information is received by the third party analyte monitoring application. According to some embodiments, the sensor type information can comprise a version number, version name, model name, model number, serial number, sensor code, or some other type of identifier indicative of the type of sensor control device worn by the monitored user associated with the connection. In some embodiments, the sensor type information can be transmitted to the third party analyte monitoring application from a trusted computer system (e.g., cloud-based server) when the connection is first established. In other embodiments, the sensor type information can be periodically requested from the trusted computer system by the third party analyte monitoring application.

Referring again to FIG. 10I, at Step 1056, the third party analyte monitoring application determines the alarm notification settings interface based on the received sensor type information. For example, at Step 1058, a first set of alarm notification settings can be displayed if it is determined that the monitored user associated with the connection is utilizing a sensor control device of a first sensor type. Similarly, at Step 1060, a second set of alarm notification settings can be displayed if it is determined that the monitored user associated with the connection is utilizing a sensor control device of a second sensor type. Furthermore, in some embodiments, if the sensor type of the monitored user associated with the connection cannot be ascertained (or, as another example, if the sensor type of the monitored user is determined to be unsupported), then a third set of alarm notification settings can be displayed. In addition, according to some embodiments, prior to displaying the notification settings interfaces for a particular monitored user (Steps 1058, 1060, or 1062), the third party analyte monitoring application can receive the monitored user's notification settings via the trusted computer system. If this is the first time the third party has viewed the notification settings for the monitored user, the third party analyte monitoring application can automatically set the notification settings using the monitored user's alarm settings. Subsequently, according to some embodiments, the third party can modify the notification settings for the monitored user within the third party analyte monitoring application, which can then save any changes going forward.

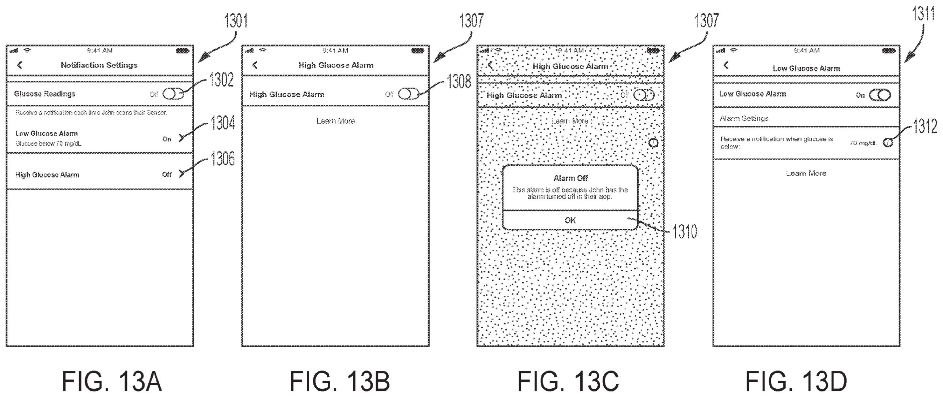

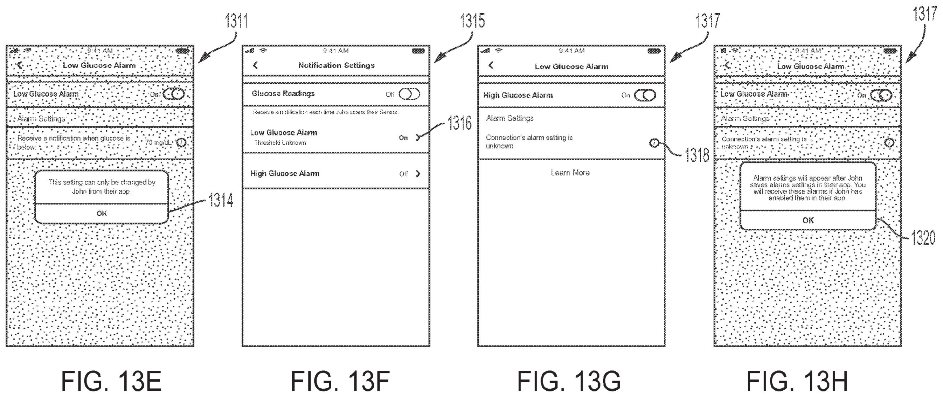

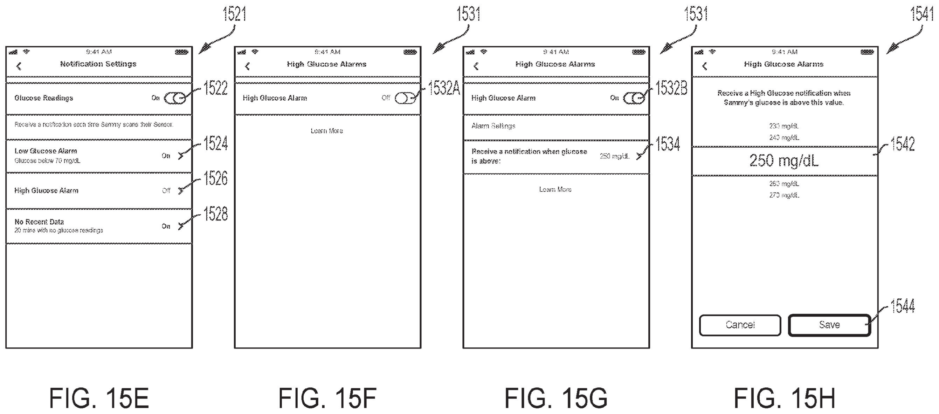

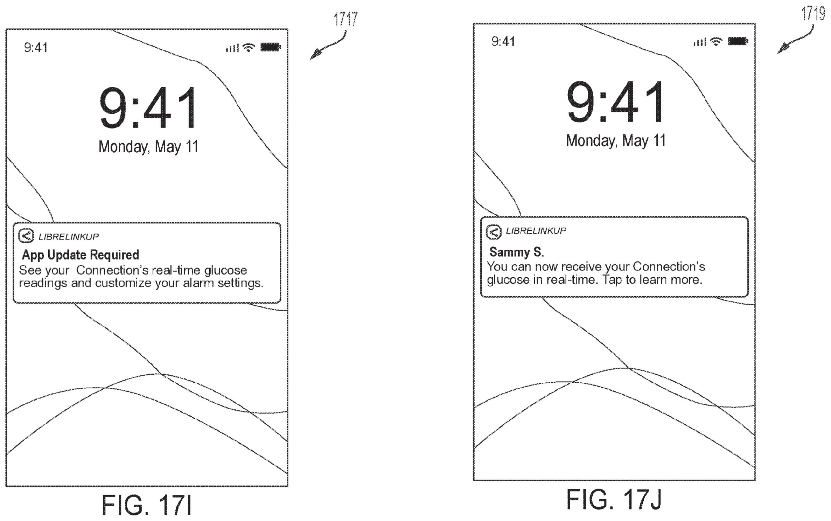

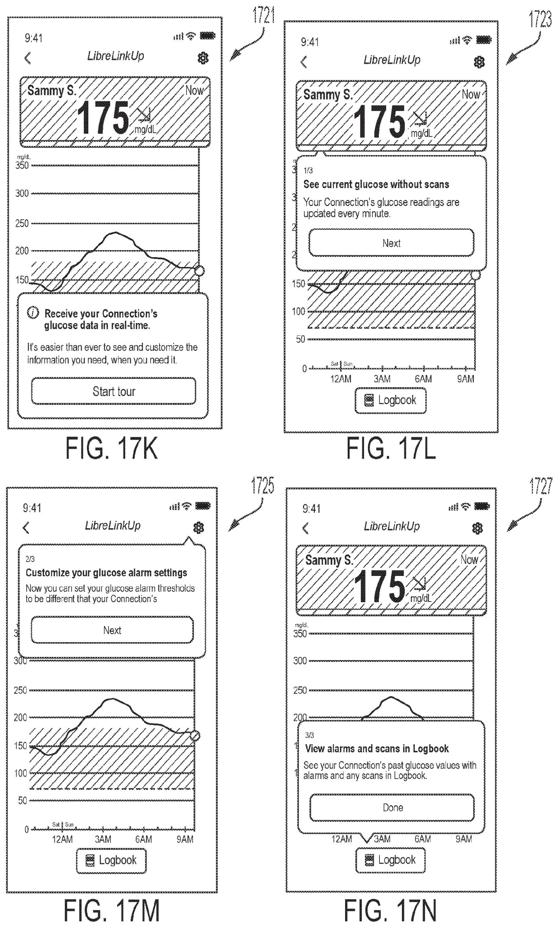

In this regard, the third party analyte monitoring application provides for flexibility as it can be configured to operate with a variety of sensor control devices and, optionally, can adopt the alarm settings of the monitored user while allowing the third party to modify them going forward. This can be useful in situations where a third party needs to support multiple connections with multiple monitored users, or where a monitored user has changed his or her sensor control device. According to another aspect of some embodiments, the third party analyte monitoring software can dynamically modify certain interfaces and settings, without user intervention, when a change is detected in the monitored user's sensor control device and/or analyte monitoring software application. By way of one non-limiting example, the third party monitoring analyte monitoring application can detect if a monitored user's sensor control device has been upgraded from a scan-based device (e.g., transmits data in response to a request or scan) to a streaming device (e.g., transmits data autonomously). In response to detecting that the sensor control device has been upgraded, a notification can be outputted to prompt the third party to update the third party analyte monitoring application (FIG. 17I). Once the third party analyte monitoring application has been updated, a notification can be outputted to inform the third party that real-time monitoring is available for the monitored user whose sensor control device has been upgraded (FIG. 17J). Furthermore, a modified set of notification settings to support the new functionality of the upgraded sensor control device can then be associated with the monitored user. For instance, if the monitored user's sensor control device is upgraded from a scan-based device to a streaming device, then the third party analyte monitoring software, which had previously displayed a first type of notification settings (e.g., FIGS. 13A to 13H), can then display a second type of notification settings (e.g., FIGS. 15E to 15H). Similarly, the logbook interface displayed can also change if the monitored user's sensor control device and/or analyte monitoring software application is modified. For example, according to one embodiment, if a monitored user's sensor control device is upgraded from a scan-based device to a streaming device, then the third party analyte monitoring software can modify the logbook associated with the monitored user from a first type of logbook (e.g., FIG. 10A) to a second type of logbook (e.g., FIG. 10E).

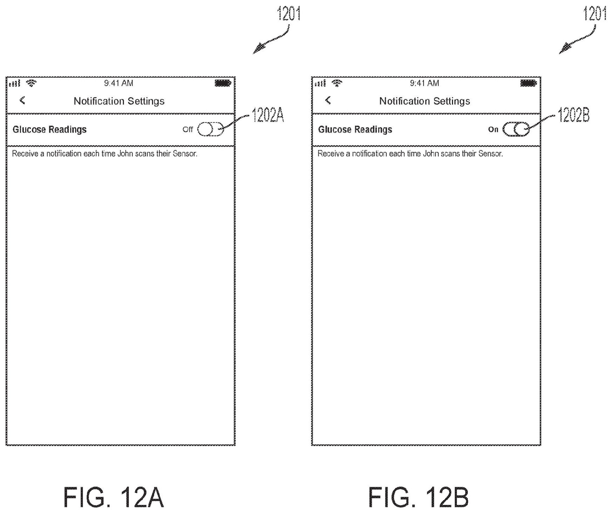

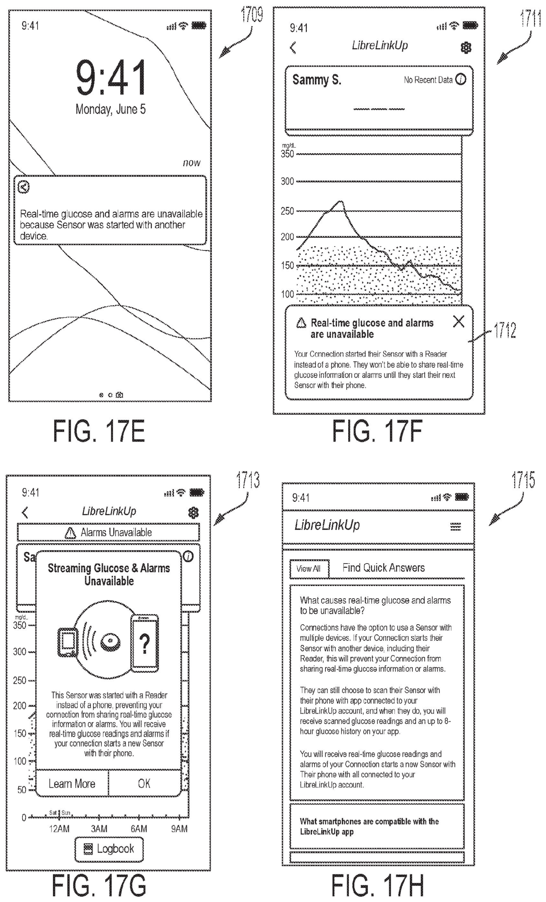

By way of another non-limiting example, the third party monitoring application can automatically detect if the monitored user's sensor control device, which had been communicatively coupled with a primary display device (e.g., the monitored user's smart phone), has been subsequently communicatively coupled with a secondary display device (e.g., a dedicated reader device) such that the sensor control device is no longer streaming data to the monitored user's primary display device. Subsequently, the third party analyte monitoring application can output an alert or notification (e.g., FIGS. 17E to 17H), and then automatically display an appropriate set of notification settings in accordance with the secondary display device. For instance, if the monitored user's sensor control device is communicatively coupled with a secondary display device, then the third party analyte monitoring software, which had previously displayed a second type of notification settings (e.g., FIGS. 13A to 13H, FIGS. 14A to 14G, or FIGS. 15E to 15H), can then display a first type of notification settings (e.g., FIGS. 12A to 12B).

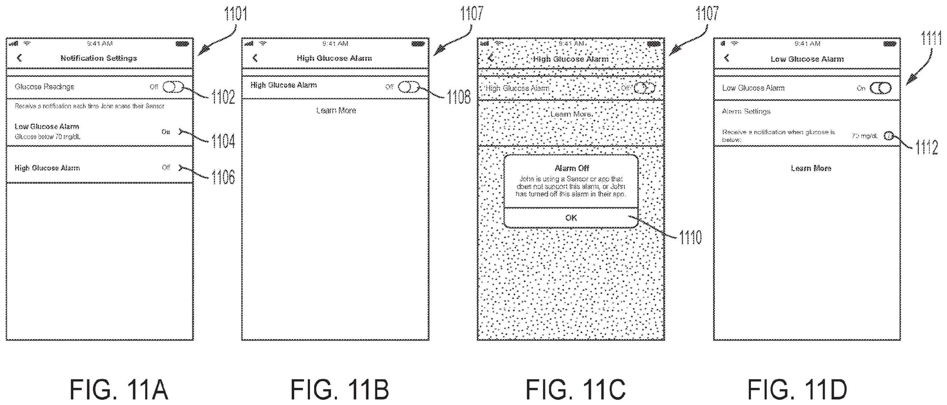

FIGS. 11A to 11H depict an example embodiment of a set of alarm notification settings interfaces for a third party analyte monitoring application. In some embodiments, these interfaces can comprise the third set of alarm notification settings described with respect to Step 1062 of method 1050 (FIG. 10I).

Referring first to FIG. 11A, alarm notification settings GUI 1101 comprises a glucose readings switch 1102 (depicted in an "off" state), a low glucose alarm setting 1104 (depicted in an "on" state), and a high glucose alarm setting 1106 (depicted in an "off" state). According to one aspect of the embodiments, the glucose readings switch, when turned on, can cause alarm notifications to be presented on the third party's secondary display device when the monitored user takes a glucose reading (e.g., scans the sensor control device with his reader device). According to another aspect of the embodiments, low glucose alarm setting 1104 can further include text that indicates a low glucose threshold value.

When the third party selects the high glucose alarm setting 1106 of GUI 1101, third party analyte monitoring application will display another alarm notification settings GUI 1107, as shown in FIG. 11B. GUI 1107 includes a high glucose alarm switch 1108 (depicted in an "off" state). According to some embodiments, if the third party attempts to toggle the high glucose alarm setting 1108 to an "on" state, a modal 1110 is displayed (as shown in FIG. 11C) to indicate that the monitored user "is using a Sensor or app that does not support this alarm," or that the monitored user "has turned off this alarm in their application." According to one aspect of the embodiments, modal 1110 can be displayed based on sensor type information relating to the type of sensor control device utilized by the monitored user associated with the connection.

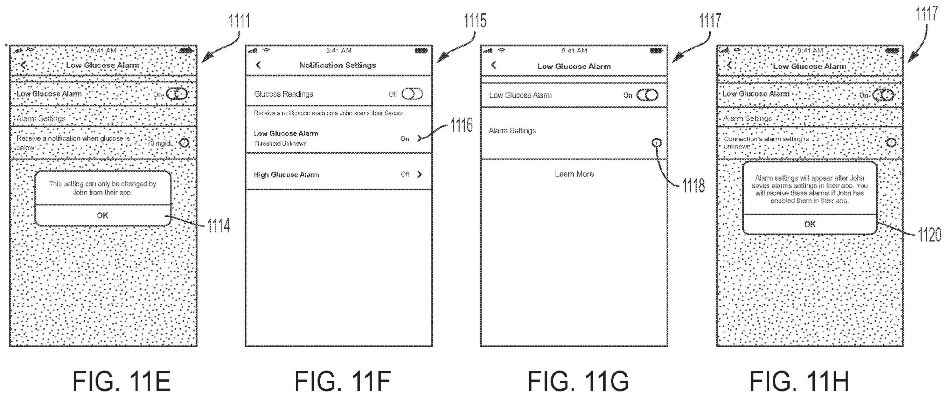

Referring back to FIG. 11A, if the third party selects the low glucose alarm setting 1104, another alarm notification settings GUI 1111 is displayed. As can be seen in FIG. 11D, GUI 1111 comprises a low glucose alarm switch (depicted in the "on" state) and a low glucose alarm threshold setting 1112 (including an informational icon). If the low glucose alarm threshold setting 1112 is selected, a modal 1114 is displayed, as shown in FIG. 11E, indicating that the setting can only be changed by the monitored user from the analyte monitoring application. Again, modal 1114 can be displayed based on sensor type information relating to the type of sensor control device utilized by the monitored user associated with the connection.

FIG. 11F to 11H depict another example embodiment of a set of alarm notification settings GUIs, where certain information regarding the monitored user's alarm settings is unknown. Referring to FIG. 11F, GUI 1115, for example, resembles GUI 1101 of FIG. 11A, except that the low glucose alarm setting 1116 indicates that the low glucose threshold is unknown. If the third party selects the low glucose alarm setting 1116, another alarm notification settings GUI 1117 is displayed. As can be seen in FIG. 11G, GUI 1117 resembles GUI 1111 of FIG. 11D, except that a textual indication 1118 that the connection's alarm setting is "unknown" is displayed (instead of a low glucose threshold setting). Similarly, if the informational icon 1118 is selected, a modal 1120 is displayed (as shown in FIG. 11H) to provide further information regarding the status of this particular alarm setting.

FIGS. 12A and 12B depict another example embodiment of a set of alarm notification settings GUIs for a third party analyte monitoring application. In some embodiments, these interfaces can comprise the first set of alarm notification settings described with respect to Step 1058 of method 1050 (FIG. 10I). Referring to FIG. 12A, alarm notification settings GUI 1201 comprises a glucose readings switch 1202A depicted in an "off" state. FIG. 12B depicts the same GUI 1201 with the glucose readings switch 1202B depicted in an "on" state. As described earlier, if the glucose readings switch 1202B is enabled, then the third party analyte monitoring application can be configured to cause alarm notifications to be presented on the third party's secondary display device when the monitored user takes a glucose reading (e.g., scans the sensor control device with his reader device).

According to one aspect of the embodiments, GUI 1201 does not include other settings (e.g., low glucose alarm, high glucose alarm, etc.) because, as indicated by sensor type information received by the third party analyte monitoring application, the sensor control device used by the monitored user associated with the connection either does not support such settings.

FIGS. 13A to 13H depict another example embodiment of a set of alarm notification settings GUIs for a third party analyte monitoring application. In several aspects, these interfaces are substantially similar to those depicted in FIGS. 11A to 11H, and can also comprise the first set of alarm notification settings described with respect to Step 1058 of method 1050 (FIG. 10I). Referring first to FIG. 13A, alarm notification settings GUI 1301 is similar to GUI 1101 (FIG. 11A), in that GUI 1301 also comprises a glucose readings switch 1302 (depicted in an "off" state), a low glucose alarm setting 1304 (depicted in an "on" state), and a high glucose alarm setting 1306 (depicted in an "off" state). Likewise, according to one aspect of the embodiments, the glucose readings switch 1302, when turned on, can cause alarm notifications to be presented on the third party's secondary display device when the monitored user takes a glucose reading (e.g., scans the sensor control device with his reader device). Similarly, according to another aspect of the embodiments, low glucose alarm setting 1304 can further include text that displays a low glucose threshold value.

When the third party selects the high glucose alarm setting 1306 of GUI 1301, third party analyte monitoring application will display another alarm notification settings GUI 1307, as shown in FIG. 13B. GUI 1307 includes a high glucose alarm switch 1308 (depicted in an "off" state). According to some embodiments, if the third party attempts to toggle the high glucose alarm setting 1308 to an "on" state, a modal 1310 is displayed to indicate that the monitored user has turned off this particular alarm via the analyte monitoring application on the monitored user's reader device. According to one aspect of the embodiments, modal 1310 can be displayed based on sensor type information relating to the type of sensor control device utilized by the monitored user associated with the connection.

Referring back to FIG. 13A, when the third party selects the low glucose alarm setting 1304, another alarm notification settings GUI 1311 is displayed. As can be seen in FIG. 13D, GUI 1311 comprises a low glucose alarm switch (depicted in the "on" state) and a low glucose alarm threshold setting 1312 (including an informational icon). If the low glucose alarm threshold setting 1312 is selected, a modal 1314 is displayed, as shown in FIG. 13E, indicating that the setting can only be changed by the monitored user from the analyte monitoring application. Again, modal 1314 can be displayed based on sensor type information relating to the type of sensor control device utilized by the monitored user associated with the connection.

FIG. 13F to 13H depict another example embodiment of a set of alarm notification settings GUIs, where certain information regarding the monitored user's alarms settings is unknown. GUI 1315, for example, resembles GUI 1301 of FIG. 13A, except that the low glucose alarm setting 1316 indicates that the low glucose threshold is unknown. If the third party selects the low glucose alarm setting 1316, another alarm notification settings GUI 1317 is displayed. As can be seen in FIG. 13G, GUI 1317 resembles GUI 1311 of FIG. 13D, except that a textual indication 1318 that the connection's alarm setting is "unknown" is displayed (instead of a low glucose threshold setting). Similarly, if the informational icon 1318 is selected, a modal 1320 is displayed to provide further information regarding the status of this particular alarm setting.

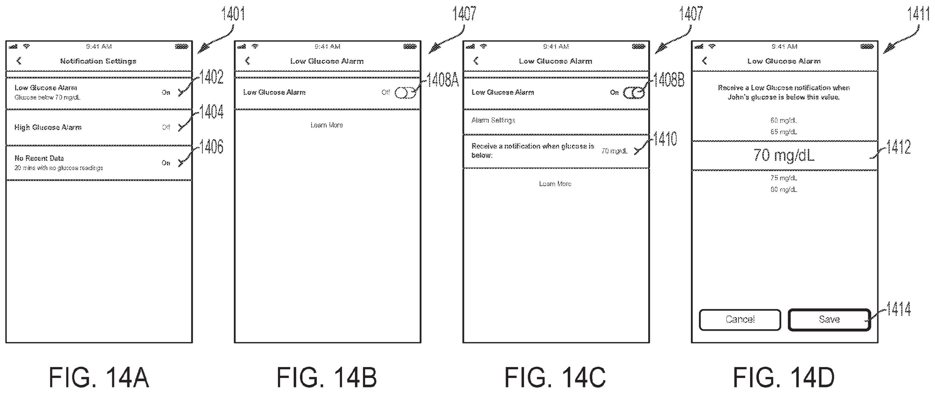

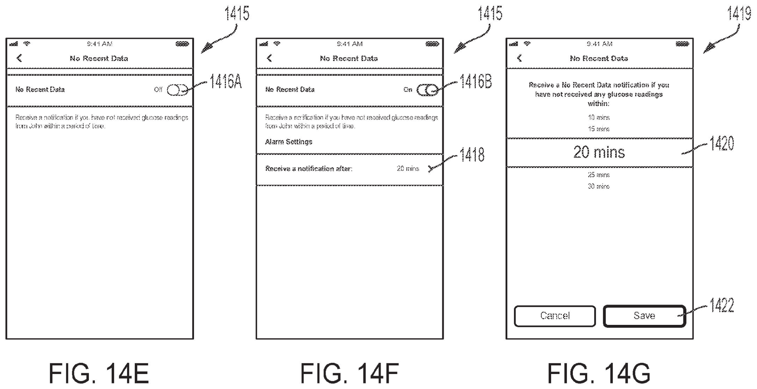

FIGS. 14A to 14G depict another example embodiment of a set of alarm notification settings GUIs for a third party analyte monitoring application. In some embodiments, these interfaces can comprise the second set of alarm notification settings described with respect to Step 1060 of method 1050 (FIG. 10I).

Referring first to FIG. 14A, alarm notification settings GUI 1410 comprises a low glucose alarm setting 1402 (depicted in an "on" state) and a high glucose alarm setting 1404 (depicted in an "off" state). Furthermore, according to one aspect of these embodiments, GUI 1410 also includes a no recent data setting 1406 (depicted in an "on" state) and does not include a glucose readings switch (as shown in FIG. 11A) because the sensor type information received by the third party analyte monitoring application indicates that the monitored user's sensor control device is configured to autonomously stream analyte data to the monitored user's reader device (e.g., via a Bluetooth or Bluetooth Low Energy protocol). According to another aspect of the embodiments, a low glucose alarm setting 1402 can include text that indicates the low glucose threshold value, and the no recent data setting 1406 can include text that indicates a threshold time elapsed since the last valid current glucose reading.

FIGS. 14B to 14D depict additional example embodiments of alarm notification settings GUIs relating to a low glucose alarm. In particular, according to some embodiments, the selection of the low glucose alarm setting 1402 (FIG. 14A) can cause display of a low glucose alarm GUI 1407. FIG. 14B shows low glucose alarm GUI 1407 with a low glucose alarm switch 1408A in an "off" state. FIG. 14C shows low glucose alarm GUI 1407 with the low glucose alarm switch 1408B in an "on" state, which also causes display of a low glucose alarm threshold setting 1410. According to some embodiments, when the low glucose alarm threshold setting 1410 is selected, a low glucose alarm threshold GUI 1411 is displayed. As can be seen in FIG. 14D, the low glucose alarm threshold GUI 1411 includes a wheel interface 1412 by which a third party can select a low glucose threshold value. Those of skill in the art will recognize that other user interface features for selecting a numeric value, such as, e.g., a pulldown menu or a free text field, can be utilized, and are fully within the scope of the present disclosure. Once the low glucose threshold value is selected, the third party can select the "Save" button 1414 to apply any changes to the low glucose alarm settings.

FIGS. 14E to 14G depict additional example embodiments of alarm notification settings GUIs relating to the no recent data alarm. In particular, according to some embodiments, the selection of the no recent data setting 1406 (FIG. 14A) can cause display of a no recent data alarm GUI 1415. FIG. 14E shows the no recent data alarm GUI 1415 with the no recent data alarm switch 1416A in an "off" state. FIG. 14F shows the no recent data alarm GUI 1415 with the no recent data alarm switch 1416B in an "on" state, which also causes display of a threshold time elapsed setting 1418 indicating a threshold time elapsed since a last valid current glucose reading was received before triggering the no recent data alarm. Furthermore, according to some embodiments, when the threshold time elapsed setting 1418 is selected, a threshold time elapsed GUI 1419 is displayed. As can be see in FIG. 14G, the threshold time elapsed GUI 1419 includes a wheel interface 1420 by which the third party can select a threshold time elapsed value. Again, those of skill in the art will recognize that other user interface features for selecting a numeric value, such as, e.g., a pulldown menu or a free text field, can be utilized, and are fully within the scope of the present disclosure. Once the threshold time elapsed value is selected, the third party can select the "Save" button 1422 to apply any changes to the no recent data settings.

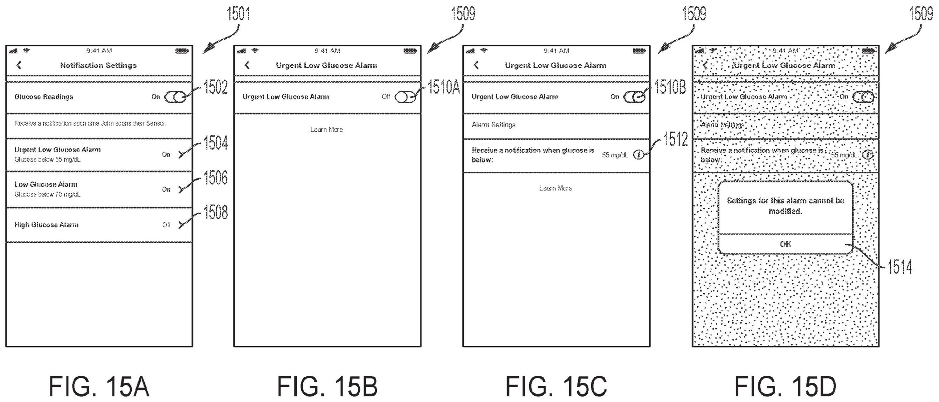

FIGS. 15A to 15D depict another example embodiment of a set of alarm notification settings interfaces for a third party analyte monitoring application. In several aspects, these interfaces are substantially similar to those depicted in FIGS. 13A to 13H, and can also comprise the first set of alarm notification settings described with respect to Step 1058 of method 1050 (FIG. 10I). Referring first to FIG. 15A, alarm notification settings GUI 1501 is similar to GUI 1301 (FIG. 13A), in that GUI 1501 also includes a glucose readings switch 1502 (depicted in an "on" state), a low glucose alarm setting 1506 (depicted in an "on" state), and a high glucose alarm setting 1508 (depicted in an "off" state). In addition, GUI 510 comprises an urgent low glucose alarm 1504 (depicted in an "on" state). According to another aspect of the embodiments, the low glucose alarm setting 1506 and urgent low glucose alarm setting 1504 can each include text that indicates their respective threshold values.

When a third party selects the urgent low glucose alarm setting 1504 of GUI 1501, third party analyte monitoring application will display an urgent low glucose alarm GUI 1509, as shown in FIGS. 15B. GUI 1509 includes an urgent low glucose alarm switch 1510A, depicted in an "off" state. FIG. 15C depicts GUI 1509 with the urgent low glucose alarm setting in an "on" state, which further causes the display of an urgent low glucose threshold value 1512 and an informational icon. If the urgent low glucose threshold value 1512 or informational icon are selected, a modal 1514 is displayed, as shown in FIG. 15D, indicating that the settings for the urgent low glucose alarm cannot be modified. Modal 1514 can be displayed based on sensor type information relating to the type of sensor control device utilized by the monitored user associated with the connection.

FIGS. 15E to 15H depict another example embodiment of a set of alarm notification settings interfaces for a third party analyte monitoring application. In several aspects, these interfaces are substantially similar to those depicted in FIGS. 14A to 14G, and can also comprise the second set of alarm notification settings described with respect to Step 1060 of method 1050 (FIG. 10I). Referring first to FIG. 15E, alarm notification settings GUI 1521 comprises a low glucose alarm setting 1524 (depicted in an "on" state), and a high glucose alarm setting 1526 (depicted in an "off" state). The low glucose alarm setting 1524 can include text that indicates the low glucose threshold value, and the no recent data setting 1528 can include text that indicates a threshold time elapsed since the last valid current glucose reading.

Furthermore, according to one aspect of these embodiments, GUI 1521 also includes both a glucose readings switch 1522 (depicted in an "on" state) and a no recent data setting 1528 because the sensor type information received by the third party analyte monitoring application indicates that the monitored user's sensor control device is configured for both: (1) scan-based or request-based transmissions of analyte data, and (2) autonomous streaming of analyte data (e.g., via a Bluetooth or Bluetooth Low Energy protocol).

FIGS. 15F to 15H depict additional example embodiments of alarm notification settings GUIs relating to a high glucose alarm. In particular, according to some embodiments, the selection of the high glucose alarm setting 1526 (FIG. 15E) can cause display of a high glucose alarm GUI 1531. FIG. 15F shows high glucose alarm GUI 1531 with a high glucose alarm switch 1532A in an "off" state. FIG. 15G shows high glucose alarm GUI 1531 with the high glucose alarm switch 1532B in an "on" state, which also causes display of a high glucose alarm threshold setting 1534. According to some embodiments, when the high glucose alarm threshold setting 1534 is selected, a high glucose alarm threshold GUI 1541 is displayed. As can be seen in FIG. 15H, the high glucose alarm threshold GUI 1541 includes a wheel interface 1542 by which a third party can select a high glucose threshold value. Those of skill in the art will recognize that other user interface features for selecting a numeric value, such as, e.g., a pulldown menu or a free text field, can be utilized, and are fully within the scope of the present disclosure. Once the high glucose threshold value is selected, the third party can select the "Save" button 1544 to apply any changes to the low glucose alarm settings. In this regard, like the previous embodiments described with respect to FIGS. 14A to 14H, a third party is able to select certain glucose alarm thresholds that are independent of the monitored user's glucose alarm thresholds.

Although the alarm notification settings GUIs and their specific elements are described in specific combinations with certain sensor control devices, those of skill in the art will appreciate that the interfaces and elements can be implemented in other combinations and permutations, and such arrangements are fully within the scope of the present disclosure.

### Example Embodiments of Alarm Notification Interfaces and Other Interfaces for Third Party Analyte Monitoring

Example embodiments of alarm notification interfaces and other types of interfaces for third party analyte monitoring will now be described. As an initial matter, it will be understood by those of skill in the art that the interfaces described herein can comprise instructions stored in memory of a secondary display device (e.g., smart phone, smart watch, wearable electronic device, dedicated receiver, etc.) belonging to a third party. In addition, those of skill in the art will also recognize that any of the alarm notification interfaces can be utilized in combination with any of the previously described alarm notification settings interfaces.

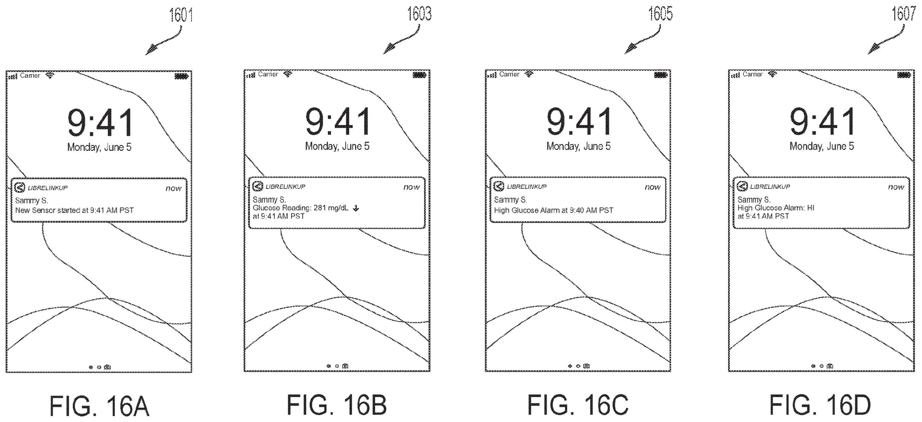

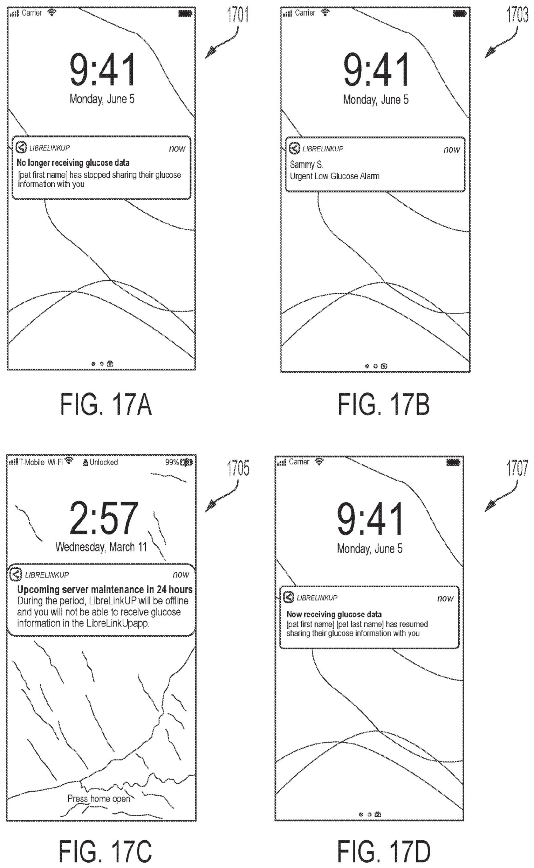

FIGS. 16A to 16H and 17A to 17O are example embodiments of alarm notification interfaces and other interfaces for third party analyte monitoring. Referring first to FIG. 16A, alarm interface 1601 depicts an example embodiment of a new sensor notification indicating that a monitored user has started a new analyte sensor at a specific time. FIG. 16B shows alarm interface 1603, which depicts an example embodiment of a glucose reading notification, which can indicate, for example, that the monitored user has scanned his or her sensor control device. Additionally, alarm interface 1603 can indicate an analyte level value (e.g., 281 mg/dL), a directional trend arrow to indicate whether the monitored user's analyte level is rising or falling, and a time stamp. FIG. 16C shows alarm interface 1605, which depicts an example embodiment of a high glucose alarm indicating that the monitored user's analyte level has exceeded a high glucose alarm threshold at a specific time. Similarly, FIG. 16D shows alarm interface 1607, which is another example embodiment of a high glucose alarm interface accompanied by an indication ("HI") that the monitored user's analyte level is above a reportable analyte level upper limit.

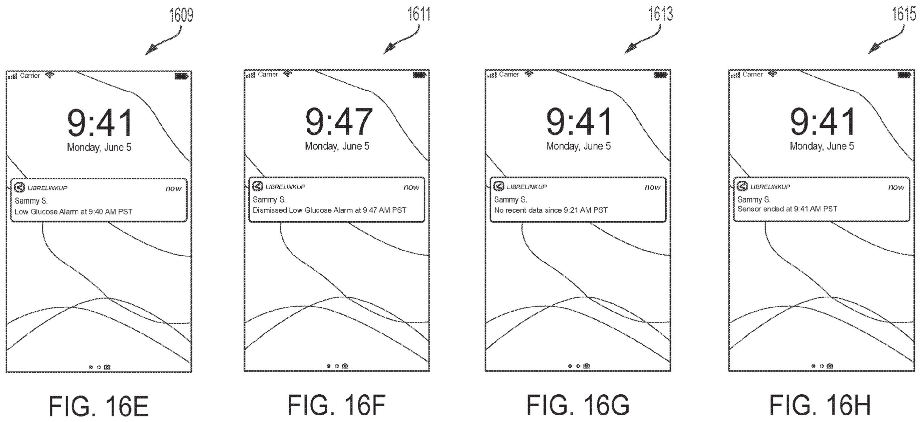

Referring next to FIG. 16E, alarm interface 1609 depicts an example embodiment of a low glucose alarm notification indicating that the monitored user's analyte level has fallen below a low glucose alarm threshold at a specific time. FIG. 16F shows alarm interface 1611, which depicts a dismissed low glucose alarm notification, indicating that the monitored user has dismissed a low glucose alarm on her reader device at a specific time. FIG. 16G shows alarm interface 1613, which depicts a no recent data notification, which can indicate that a valid current analyte level has not been received since a specific time. In some embodiments, for example, the no recent data notification can indicate that the third party analyte monitoring application on the secondary display device has not received a valid current analyte level reading from the trusted computer system. In other embodiments, the no recent data notification can indicate that the analyte monitoring application on the monitored user's reader device has not received a valid current analyte level reading from the sensor control device. In still other embodiments, the no recent data notification can indicate either or both of the aforementioned conditions, or any other condition that would prevent the third party analyte monitoring application from receiving a current valid analyte level reading.

Referring next to FIG. 16H, alarm interface 1615 depicts an example embodiment of a sensor ended alarm notification, which can indicate that the monitored user's sensor control device has expired. In some embodiments, the sensor ended notification can also indicate if the monitored user's sensor control device has been terminated for other reasons, such as, for example, a sensor fault condition.

According to an aspect of the embodiments depicted in FIGS. 16A to 16H, each of the alarm notification interfaces can be configured such that touching or tapping on the alarm notification area can cause the third party analyte monitoring application to come into the foreground and display the connection interface associated with the alarm notification. According to another aspect of the embodiments, any or all of the alarm notification interfaces described herein can be displayed without a time stamp.

Turning next to FIG. 17A, alarm interface 1701 depicts an example embodiment of a notification interface 1701 indicating that a monitored user has stopped sharing analyte information with the third party. According to one aspect of the embodiments, alarm interface 1701 can be displayed in response to the third party analyte monitoring application receiving an indication that the monitored user has stopped sharing his or her analyte information with the third party. In some embodiments, for example, the indication can be generated when the monitored user selects the Stop Sharing link 428 in the monitored user's analyte monitoring application. See, e.g., FIG. 4H. In other embodiments, the indication can be generated when the monitored user selects the remove link 416 in the monitored user's analyte monitoring application. See, e.g., FIG. 4C. In other embodiments, the indication can be generated when the monitored user deletes his or her account.

FIG. 17B is an example embodiment of an urgent low glucose alarm notification interface 1703 for a third party analyte monitoring application. According to one aspect of the embodiments, the urgent low glucose alarm notification interface 1703 can implemented with the alarm notification settings interfaces described with respect to FIGS. 15A to 15D.

FIG. 17C is an example embodiment of a maintenance warning notification interface 1705 for a third party analyte monitoring application. According to one aspect of the embodiments, the maintenance warning notification interface 1705 can be initiated and updated by the trusted computer system (e.g., a cloud-based server).

FIG. 17D is an example embodiment of a notification interface 1707 indicating that a monitored user has resumed sharing analyte information with the third party. Similar to embodiments described earlier, depending on the user's particular country or market, notification interface 1707 can be displayed with the patient's first name followed by the patient's last name, the patient's first name followed by an initial of the patient's last name, or the patient's last name followed by the patient's first name.

FIG. 17E is an example embodiment of a notification interface 1709 in the form of an alert indicating that real-time glucose and alarms are unavailable for a monitored user. This alert can be displayed when the third party analyte monitoring application has detected that the monitored user has communicatively coupled their sensor control device with a secondary display device, such that the monitored user's sensor control device is no longer streaming data to the primary display device.

FIG. 17F is an example of a third party analyte monitoring application interface 1711 comprising an in-app notification 1712 indicating that real-time glucose and alarms are unavailable for a monitored user. The in-app notification 1712 can be displayed when the third party analyte monitoring application has detected that the monitored user has communicatively coupled their sensor control device with a secondary display device, such that the monitored user's sensor control device is no longer streaming data to the primary display device.

FIGS. 17G and 17H are examples of interfaces for a third party monitoring application comprising an in-app modal 1713 and an informational modal 1715 for indicating that real-time glucose and alarms are unavailable for a monitored user.

FIG. 17I is an example embodiment of a notification interface 1717 for prompting a third party to update their third party analyte monitoring application. According to some embodiments, this notification can be displayed after it is determined that the monitored user's sensor control device and/or analyte monitoring software application has been modified.

FIG. 17J is an example embodiment of a notification interface 1719 for notifying a third party that a monitored user's real-time glucose is available. According to some embodiments, this notification can be displayed after it is determined that the monitored user's sensor control device and/or analyte monitoring software application has been modified, and the third party analyte monitoring application has been updated.

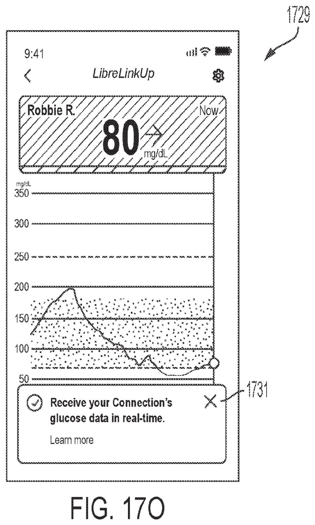

FIGS. 17K to 17N are example embodiments of tutorial interfaces 1721, 1723, 1725, and 1727, which can be displayed in a connection interface of the third party analyte monitoring application for a monitored user after the monitored user's sensor control device and/or analyte monitoring software application is upgraded. According to some embodiments, tutorial interfaces 1721, 1723, 1725, and 1727 can be displayed only during the first time a monitored user's sensor control device and/or analyte monitoring software application are upgraded. Subsequently, as shown in FIG. 17O, when another monitored user's sensor control device is upgraded, only a dismissible reminder 1731 is shown, and the third party need not be presented with the tutorial interfaces again. However, if the third party wishes to review the tutorial interfaces again, she can touch the "Learn More" link shown in FIG. 17O to launch tutorial interfaces 1721, 1723, 1725, and 1727. In other embodiments, tutorial interfaces 1721, 1723, 1725, and 1727 can be displayed every time a monitored user's sensor control device and/or analyte monitoring software application is upgraded.

It should be noted that those of skill in the art will appreciate that the aforementioned alarm notification interfaces are meant to be merely illustrative, and in no way are intended to comprise an exhaustive list. Further details regarding alarm interfaces for analyte monitoring can be found in U.S. Publ. No. 2021/0282672A1, U.S. Publ. No. 2021/0378601A1 and U.S. Patent Appl. Serial No. 17/478,447, all of which are hereby incorporated by reference in their entireties for all purposes.

### Example Embodiments of Additional GUIs for Third Party Analyte Monitoring

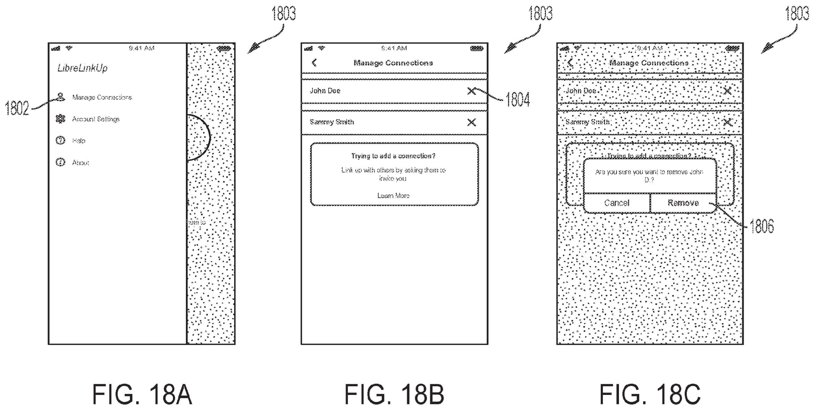

Example embodiments of additional GUIs for third party analyte monitoring will now be described. Referring first to FIG. 18A, a menu GUI 1801 for use with a third party analyte monitoring application is depicted. In some embodiments, the menu interface 1801 can comprise a plurality of selectable options including, but not limited to, a manage connections option 1802, an account settings option, a help option, and an about the software option. If the manage connection option 1802 is selected, a manage connections GUI 1803 is then displayed, as shown in FIG. 18B. According to one aspect of the embodiments, the manage connections GUI 1803 can include a listing of one or more connections (if any), with each connection listed by the name of the monitored user. In some embodiments, each connection can also include a selectable icon 1804 for removing the connection. According to another aspect of the embodiments, if the selectable icon 1804 is pressed, then a confirmation modal 1806 is displaying to prompt the third party for confirmation prior to removing the connection.

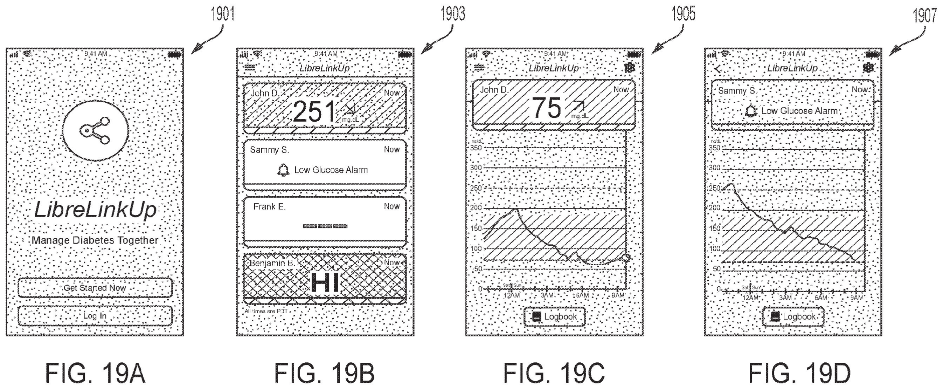

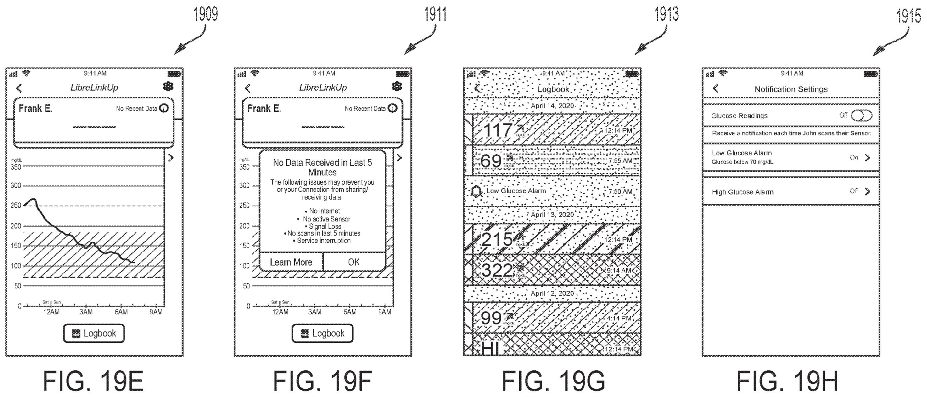

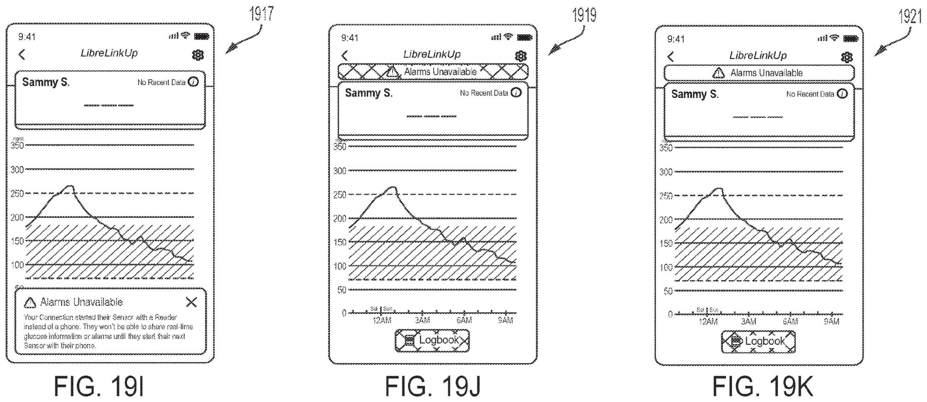

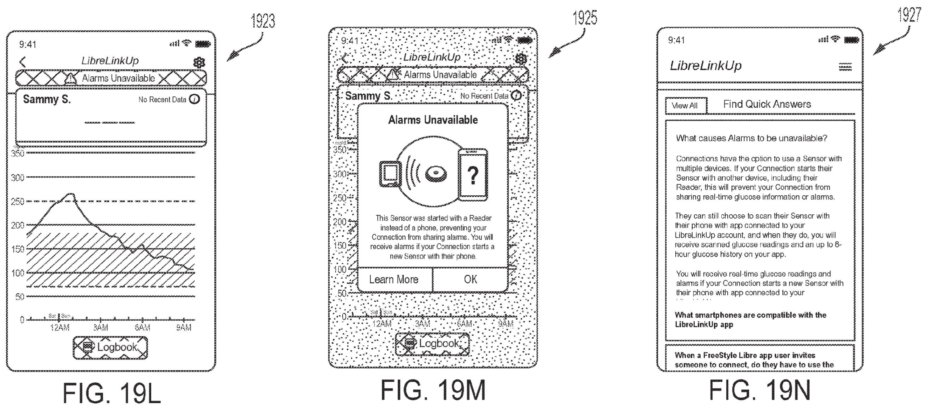

Turning to FIGS. 19A to 19N, example embodiments of GUIs relating to an enhanced visibility mode (also referred to as "dark mode") will now be described. In certain environments, it may be desirable to enhance the display of the third party's secondary display device for better visibility with respect to many of the interfaces described herein. For example, certain color shadings in a chart or graph may be difficult to see in a low light setting due to a lack of contrast between the colors. By way of another example, interfaces can be modified to utilize certain background colors which may provide less strain on the eyes in a low light setting.

FIG. 19A, for example, depicts an initial interface 1901 of the third party analyte monitoring application in an enhanced visibility mode, which is similar to the initial interface 501 of FIG. 5A. As another example, FIG. 19B depicts a multiple connections interface 1903 in an enhanced visibility mode, which is similar to the multiple connections interface 801 of FIG. 8A. Likewise, FIGS. 19C to 19F depict connection interfaces 1905, 1907, 1909, 1911, FIG. 19G depicts a logbook interface 1913, and FIG. 19H depicts an alarm notification settings interface 1915, all of which are shown in an enhanced visibility mode.

FIGS. 19I to 19N depict example embodiments of interfaces 1917, 1919, 1921, 1923, 1925, and 1927 of the third party analyte monitoring application in an enhanced visibility mode. According to one aspect of the embodiments, interfaces 1917, 1919, 1921, 1923, 1925, and 1927 are analogous to interfaces 1709, 1711, 1713, and 1715, as described with respect to FIGS. 17E to 17H, in the sense that the interfaces depict alerts or notifications to the third party that real-time glucose data and alarms are unavailable for the monitored user. According to another aspect of the embodiments, interfaces 1917, 1919, 1921, 1923, 1925, and 1927 can be displayed when the third party analyte monitoring application has detected that the monitored user has communicatively coupled their sensor control device with a secondary display device, such that the monitored user's sensor control device is no longer streaming data to the primary display device.

Furthermore, in some embodiments, the enhanced visibility mode can be enabled through a user-configurable setting of the operating system of the display device (e.g., iOS, Android). In other embodiments, enhanced visibility mode can be a user-configurable setting within the third party analyte monitoring application (e.g., an in-app setting). In still other embodiments, the enhanced visibility mode can be enabled automatically in response to the detection of light above or below a certain predetermined activation threshold by a light sensor (e.g., photoelectric devices, photo sensors, phototransistors, photoresistors, and/or photodiodes). In still other embodiments, the enhanced visibility mode can be enabled/disabled according to a predetermined time schedule (e.g., 6:00 PM to 6:00 AM) or according to a seasonal time schedule (e.g., sunset to sunrise). Those of skill in the art will understand that other activation mechanisms can be utilized and fully within the scope of the present disclosure. Further details regarding enhanced visibility mode can be found in U.S. Patent Appl. Serial No. 17/478,447, which is hereby incorporated by reference in its entirety for all purposes.

Digital and graphical user interfaces for third party analyte monitoring applications are provided. For example, disclosed herein are various embodiments of methods, systems, and interfaces for connection interfaces, alarm notification settings interfaces, and logbook interfaces in a third party analyte monitoring application. In addition, various embodiments of interface enhancements are described, including enhanced visibility mode and interfaces for setting up a third party analyte monitoring application, among other embodiments.

It will be understood by those of skill in the art that any of the GUIs (or portions thereof) described herein, are meant to be illustrative only, and that the individual elements, or any combination of elements, depicted and/or described for a particular embodiment or figure are freely combinable with any other element, or any combination of other elements, depicted and/or described with respect to any of the other embodiments.

It should also be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art.

While the embodiments are susceptible to various modifications and alternative forms, specific examples thereof have been shown in the drawings and are herein described in detail. It should be understood, however, that these embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the inventive scope of the claims by features, functions, steps, or elements that are not within that scope.

### Clauses

Exemplary embodiments are set out in the following numbered clauses.
Clause 1. A system for third party analyte monitoring, the system comprising:
   a sensor control device comprising an analyte sensor, wherein at least a portion of the analyte sensor is configured to be in fluid contact with a bodily fluid of a monitored user;
   a first reader device configured to wirelessly receive data indicative of an analyte level of the monitored user from the sensor control device, wherein the first reader device is further configured to send the data indicative of the analyte level to a trusted computer system;
   a secondary display device, comprising:
      wireless communication circuitry configured to receive, from the trusted computer system, sensor type information of the sensor control device and the data indicative of the analyte level of the monitored user,
      one or more processors coupled with a memory, the memory storing a third party analyte monitoring application that, when executed by the one or more processors, cause the one or more processors to display one or more connection interfaces reflecting the data indicative of the analyte level of the monitored user.
Clause 2. The system of clause 1, wherein the first reader device is a smart phone.
Clause 3. The system of clause 1 or 2, wherein the secondary display device is a smart phone.
Clause 4. The system of any of clauses 1-3, wherein the one or more connection interfaces reflecting the data indicative of the analyte level of the monitored user includes a first connection interface comprising a profile card section and an analyte graph section.
Clause 5. The system of clause 4, wherein the profile card section includes a name of the monitored user.
Clause 6. The system of clause 4 or 5, wherein the profile card section includes an analyte level value and a directional trend arrow based on the data indicative of the analyte level of the monitored user.
Clause 7. The system of any of clauses 4-6, wherein the profile card section comprises a background color indicative of whether a current analyte level is within a target analyte range.
Clause 8. The system of any of clauses 4-7, wherein the analyte graph section comprises an analyte trend line.
Clause 9. The system of any of clauses 4-8, wherein the analyte graph section comprises one or more lines indicative of a low glucose alarm threshold or a high glucose alarm threshold.
Clause 10. The system of any of clauses 4-9, wherein the secondary display device further comprises a touchscreen, and wherein the third party analyte monitoring application, when executed by the one or more processors, causes the one or more processors to:
   receive input from the touchscreen corresponding to a selected point on the analyte graph section, and
   update the profile card section based on the received input from the touchscreen.
Clause 11. The system of any of clauses 4-10, wherein the secondary display device further comprises a touchscreen, and wherein the third party analyte monitoring application, when executed by the one or more processors, further causes the one or more processors to:
   receive input from the touchscreen corresponding to a pulldown gesture, and
   update the profile card section and the analyte graph section based on the received input.
Clause 12. The system of any of clauses 4-11, wherein the profile card section and the analyte graph section are automatically updated at a predetermined frequency based on the data indicative of the analyte level of the monitored user.
Clause 13. The system of any of clauses 4-12, wherein the monitored user is a first monitored user, wherein the one or more connection interfaces includes a multiple connections interface comprising one or more profile card sections, and wherein the one or more profile card sections includes a first profile card section associated with the first monitored user.
Clause 14. The system of clause 13, wherein the one or more profile card sections further includes a second profile card section associated with a second monitored user.
Clause 15. The system of clause 14, wherein the first profile card section is configured to display a first analyte level value based on the data indicative of the analyte level of the first monitored user, and wherein the second profile card section is configured to display a second analyte level value based on data indicative of an analyte of the second monitored user.
Clause 16. The system of any of clauses 1-15, further comprising the trusted computer system, wherein the trusted computer system comprises a cloud-based server.
Clause 17. The system of any of clauses 1-16, wherein the sensor control device is configured to wirelessly transmit the data indicative of the analyte level of the monitored user to the first reader device according to a Bluetooth or Bluetooth Low Energy communication protocol.
Clause 18. The system of any of clauses 1-17, wherein the sensor control device is configured to wirelessly transmit the data indicative of the analyte level of the monitored user to the first reader device according to a Near Field Communication protocol
Clause 19. The system of any of clauses 1-18, wherein the wireless communication circuitry of the secondary display device is configured to communicate with the trusted computer system according to an 802.11x or cellular communication protocol.
Clause 20. The system of any of clauses 1-19, wherein first reader device is configured to wirelessly communicate with the sensor control device according to a first wireless communication protocol, and wherein the first reader device is further configured to wirelessly communicate with the trusted computer system according to a second wireless communication protocol that is different from the first wireless communication protocol.
Clause 21. A system for third party analyte monitoring, the system comprising:
   a sensor control device comprising an analyte sensor, wherein at least a portion of the analyte sensor is configured to be in fluid contact with a bodily fluid of a monitored user;
   a first reader device configured to wirelessly receive data indicative of an analyte level of the monitored user from the sensor control device, wherein the first reader device is further configured to send the data indicative of the analyte level to a trusted computer system;
   a secondary display device, comprising:
      wireless communication circuitry configured to receive, from the trusted computer system, sensor type information of the sensor control device and the data indicative of the analyte level of the monitored user,
      one or more processors coupled with a memory, the memory storing a third party analyte monitoring application that, when executed by the one or more processors, cause the one or more processors to display one or more alarm notification settings interfaces based on the sensor type information.
Clause 22. The system of clause 21, wherein the first reader device is a smart phone.
Clause 23. The system of clauses 21 or 22, wherein the secondary display device is a smart phone.
Clause 24. The system of any of clauses 21-23, wherein the third party analyte monitoring application, when executed by the one or more processors, further causes the one or more processors to:
   determine a type of sensor control device based on the received sensor type information,
   in response to a determination that the type of sensor control device is a first sensor type, displaying a first set of alarm notification settings interfaces, and
   in response to a determination that the type of sensor control device is a second sensor type, displaying a second set of alarm notification settings interfaces,
   wherein the first sensor type is different from the second sensor type, and wherein the first set of alarm notification settings interfaces is different from the second set of alarm notification settings interfaces.
Clause 25. The system of clause 24, wherein the first set of alarm notification settings interfaces includes a glucose readings switch.
Clause 26. The system of clause 25, wherein the second set of alarm notification settings interfaces does not include a glucose readings switch.
Clause 27. The system of any of clauses 24-26, wherein the second set of alarm notification settings interfaces includes a no recent data setting.
Clause 28. The system of clause 27, wherein the first set of alarm notification settings interfaces does not include a no recent data setting.
Clause 29. The system of any of clauses 24-28, wherein the first set of alarm notification settings interfaces includes an urgent low glucose alarm setting.
Clause 30. The system of clause 29, wherein the first set of alarm notification settings interfaces further includes an urgent low glucose threshold value.
Clause 31. The system of clause 30, wherein the urgent low glucose threshold value is a non-modifiable setting.
Clause 32. The system of any of clauses 24-31, wherein the first set of alarm notification settings interfaces and the second set of alarm notification settings interfaces each include a low glucose alarm setting.
Clause 33. The system of any of clauses 24-32, wherein the first set of alarm notification settings interfaces and the second set of alarm notification settings interfaces each include a high glucose alarm setting.
Clause 34. The system of any of clauses 24-33, wherein the third party analyte monitoring application, when executed by the one or more processors, further causes the one or more processors to:
   in response to a determination that the type of sensor control device is an unknown sensor type or an unsupported sensor type, displaying a third set of alarm notification settings interfaces,
   wherein the third set of alarm notification settings interfaces is different from the first set of alarm notification settings interfaces and the second set of alarm notification settings interfaces.
Clause 35. The system of clause 34, wherein the third set of alarm notification setting interfaces includes a non-modifiable low glucose threshold setting or a non-modifiable high glucose threshold setting.
Clause 36. The system of any of clauses 21-35, further comprising the trusted computer system, wherein the trusted computer system comprises a cloud-based server.
Clause 37. The system of any of clauses 21-36, wherein the sensor control device is configured to wirelessly transmit the data indicative of the analyte level of the monitored user to the first reader device according to a Bluetooth or Bluetooth Low Energy communication protocol.
Clause 38. The system of any of clauses 21-37, wherein the sensor control device is configured to wirelessly transmit the data indicative of the analyte level of the monitored user to the first reader device according to a Near Field Communication protocol
Clause 39. The system of any of clauses 21-38, wherein the wireless communication circuitry of the secondary display device is configured to communicate with the trusted computer system according to an 802.11x or cellular communication protocol.
Clause 40. The system of any of clauses 21-39, wherein first reader device is configured to wirelessly communicate with the sensor control device according to a first wireless communication protocol, and wherein the first reader device is further configured to wirelessly communicate with the trusted computer system according to a second wireless communication protocol that is different from the first wireless communication protocol.
Clause 41. A system for third party analyte monitoring, the system comprising:
   a sensor control device comprising an analyte sensor, wherein at least a portion of the analyte sensor is configured to be in fluid contact with a bodily fluid of a monitored user;
   a first reader device configured to wirelessly receive data indicative of an analyte level of the monitored user from the sensor control device, wherein the first reader device is further configured to send the data indicative of the analyte level to a trusted computer system;
   a secondary display device, comprising:
      wireless communication circuitry configured to receive, from the trusted computer system, sensor type information of the sensor control device and the data indicative of the analyte level of the monitored user,
      one or more processors coupled with a memory, the memory storing a third party analyte monitoring application that, when executed by the one or more processors, cause the one or more processors to display a logbook interface reflecting the data indicative of the analyte level of the monitored user.
Clause 42. The system of clause 41, wherein the first reader device is a smart phone.
Clause 43. The system of clause 41 or 42, wherein the secondary display device is a smart phone.
Clause 44. The system of any of clauses 41-43, wherein the logbook interface comprises a plurality of logbook entries for a predetermined period of time.
Clause 45. The system of clause 44, wherein the plurality of logbook entries is grouped by date.
Clause 46. The system of clause 44 or 45, wherein the plurality of logbook entries includes a first logbook entry, wherein the first logbook entry includes an analyte level value, a directional trend arrow, and a time stamp.
Clause 47. The system of clause 46, wherein the plurality of logbook entries further includes a second logbook entry, wherein the second logbook entry comprises a textual indication that an analyte level of the monitored user is above a reportable analyte level upper limit.
Clause 48. The system of clause 46 or 47, wherein the plurality of logbook entries further includes a second logbook entry, wherein the second logbook entry comprises a low glucose alarm.
Clause 49. The system of any of clauses 46-48, wherein the first logbook entry further includes an alarm icon.
Clause 50. The system of any of clauses 46-49, wherein the plurality of logbook entries includes a second logbook entry, wherein the second logbook entry comprises an entry manually input by the monitored user.
Clause 51. The system of any of clauses 41-50, further comprising the trusted computer system, wherein the trusted computer system comprises a cloud-based server.
Clause 52. The system of any of clauses 41-51, wherein the sensor control device is configured to wirelessly transmit the data indicative of the analyte level of the monitored user to the first reader device according to a Bluetooth or Bluetooth Low Energy communication protocol.
Clause 53. The system of any of clauses 41-52, wherein the sensor control device is configured to wirelessly transmit the data indicative of the analyte level of the monitored user to the first reader device according to a Near Field Communication protocol
Clause 54. The system of any of clauses 41-53, wherein the wireless communication circuitry of the secondary display device is configured to communicate with the trusted computer system according to an 802.11x or cellular communication protocol.
Clause 55. The system of any of clauses 41-54, wherein first reader device is configured to wirelessly communicate with the sensor control device according to a first wireless communication protocol, and wherein the first reader device is further configured to wirelessly communicate with the trusted computer system according to a second wireless communication protocol that is different from the first wireless communication protocol.

## Claims

1. A system (100) for third party analyte monitoring, the system comprising:
a sensor control device (102) comprising an analyte sensor (104), wherein at least a portion of the analyte sensor is configured to be in fluid contact with a bodily fluid of a monitored user;
a first reader device (120-1) configured to wirelessly receive data indicative of an analyte level of the monitored user from the sensor control device, wherein the first reader device is further configured to send the data indicative of the analyte level to a trusted computer system (180);
a secondary display device (120-2), comprising:
wireless communication circuitry (232) configured to receive, from the trusted computer system, sensor type information of the sensor control device and the data indicative of the analyte level of the monitored user,
one or more processors (224) coupled with a memory (225), the memory storing a third party analyte monitoring application that, when executed by the one or more processors, cause the one or more processors to display one or more alarm notification settings interfaces based on the sensor type information.

2. The system (100) of claim 1, wherein the first reader device (120-1) is a smart phone, and/or wherein the secondary display device (120-2) is a smart phone.

3. The system (100) of any preceding claim, wherein the third party analyte monitoring application, when executed by the one or more processors (224), further causes the one or more processors to:
determine a type of sensor control device (102) based on the received sensor type information,
in response to a determination that the type of sensor control device is a first sensor type, displaying a first set of alarm notification settings interfaces, and
in response to a determination that the type of sensor control device is a second sensor type, displaying a second set of alarm notification settings interfaces,
wherein the first sensor type is different from the second sensor type, and wherein the first set of alarm notification settings interfaces is different from the second set of alarm notification settings interfaces.

4. The system (100) of claim 3, wherein the first set of alarm notification settings interfaces includes a glucose readings switch, optionally wherein the second set of alarm notification settings interfaces does not include a glucose readings switch.

5. The system (100) of claims 3 or 4, wherein the second set of alarm notification settings interfaces includes a no recent data setting, optionally wherein the first set of alarm notification settings interfaces does not include a no recent data setting.

6. The system (100) of any of claims 3-5, wherein the first set of alarm notification settings interfaces includes an urgent low glucose alarm setting.

7. The system (100) of claim 6, wherein the first set of alarm notification settings interfaces further includes an urgent low glucose threshold value, optionally wherein the urgent low glucose threshold value is a non-modifiable setting.

8. The system (100) of any preceding claim, wherein the first set of alarm notification settings interfaces and the second set of alarm notification settings interfaces each include a low glucose alarm setting.

9. The system (100) of any preceding claim, wherein the first set of alarm notification settings interfaces and the second set of alarm notification settings interfaces each include a high glucose alarm setting.

10. The system (100) of any preceding claim, wherein the third party analyte monitoring application, when executed by the one or more processors (224), further causes the one or more processors to:
in response to a determination that the type of sensor control device (102) is an unknown sensor type or an unsupported sensor type, displaying a third set of alarm notification settings interfaces,
wherein the third set of alarm notification settings interfaces is different from the first set of alarm notification settings interfaces and the second set of alarm notification settings interfaces, optionally wherein the third set of alarm notification setting interfaces includes a non-modifiable low glucose threshold setting or a non-modifiable high glucose threshold setting.

11. The system (100) of any preceding claim, further comprising the trusted computer system (180), wherein the trusted computer system comprises a cloud-based server.

12. The system (100) of any preceding claim, wherein the sensor control device (102) is configured to wirelessly transmit the data indicative of the analyte level of the monitored user to the first reader device (120-1) according to a Bluetooth or Bluetooth Low Energy communication protocol.

13. The system (100) of any preceding claim, wherein the sensor control device (102) is configured to wirelessly transmit the data indicative of the analyte level of the monitored user to the first reader device (120-1) according to a Near Field Communication protocol.

14. The system (100) of any preceding claim, wherein the wireless communication circuitry (232) of the secondary display device (120-2) is configured to communicate with the trusted computer system (180) according to an 802.11x or cellular communication protocol.

15. The system (100) of any preceding claim, wherein first reader device (120-1) is configured to wirelessly communicate with the sensor control device (102) according to a first wireless communication protocol, and wherein the first reader device (120-1) is further configured to wirelessly communicate with the trusted computer system (180) according to a second wireless communication protocol that is different from the first wireless communication protocol.
